# EUROPEAN PATENT SPECIFICATION

(11) **EP 4 567 715 B1**
(45) Date of publication and mention of the grant of the patent: **13.05.2026**
(21) Application number: 24216714.6
(22) Date of filing: 02.12.2024
(51) Int. Cl.: G06T 5/60, G06T 5/70, G16H 30/40, G06T 7/00

(54) **GENERATING SYNTHETIC REPRESENTATIONS**
ERZEUGUNG SYNTHETISCHER DARSTELLUNGEN
GÉNÉRATION DE REPRÉSENTATIONS SYNTHÉTIQUES

(30) Priority: 06.12.2023 EP 23214619; 31.05.2024 EP 24179377
(43) Date of publication of application: 11.06.2025
(73) Proprietor: Bayer Aktiengesellschaft, 51373 Leverkusen (DE)
(72) Inventor: Mohammadi, Seyed Sadegh, 50968 Köln (DE); Montalt Tordera, Javier, 46014 Valencia (ES); Prokop, Jakub, 02-326 Warschau (PL)
(74) Representative: BIP Patents

(56) References cited:
- WO-A1-2014/036473
- US-A1- 2023 377 099
- AMIRHOSSEIN KAZEROUNI ET AL: "Diffusion Models for Medical Image Analysis: A Comprehensive Survey", ARXIV.ORG, CORNELL UNIVERSITY LIBRARY, 201 OLIN LIBRARY CORNELL UNIVERSITY ITHACA, NY 14853, 14 November 2022 (2022-11-14), XP091368786
- DAYARATHNA SANUWANI ET AL: "Deep learning based synthesis of MRI, CT and PET: Review and analysis", MEDICAL IMAGE ANALYSIS, OXFORD UNIVERSITY PRESS, OXOFRD, GB, vol. 92, 1 December 2023 (2023-12-01), XP087448589, ISSN: 1361-8415, [retrieved on 20231201], DOI: 10.1016/J.MEDIA.2023.103046

## Description

### FIELD OF THE DISCLOSURE

Systems, methods, and computer programs disclosed herein relate to generating synthetic representations, such as synthetic radiologic images.

### BACKGROUND

Artificial intelligence is increasingly finding its way into medicine. Machine learning models are being used not only to recognize signs of disease in medical images of the human or animal body (see, for example, WO2018202541A1, WO2020229152A1), but also increasingly to generate synthetic (artificial) medical images.

For example, WO2019/074938A1 and WO2022184297A1 describe methods for generating a synthetic radiological image showing an examination area of an examination object after application of a standard amount of a contrast agent, although only a smaller amount of contrast agent than the standard amount was applied. The standard amount is the amount recommended by the manufacturer and/or distributor of the contrast agent and/or the amount approved by a regulatory authority and/or the amount listed in a package insert for the contrast agent. The methods described in WO2019/074938A1 and WO2022184297A can therefore be used to reduce the amount of contrast agent.

The machine learning models disclosed in the cited publications are or include convolutional neural networks. Such machine learning models can be difficult to train, and they often require extensive tuning of hyperparameters; such models can be unstable and sometimes produce images that are not realistic or do not match the training data. Overfitting is a frequently observed problem (see, e.g., P. Thanapol et al.: Reducing Overfitting and Improving Generalization in Training Convolutional Neural Network (CNN) under Limited Sample Sizes in Image Recognition, 2020, 5th International Conference on Information Technology (InCIT), pp. 300-305, doi: 10.1109/InCIT50588.2020.9310787.

### SUMMARY

These problems are addressed by the subject matter of the independent claims of the present disclosure. Exemplary embodiments are defined in the dependent claims, the description, and the drawings.

In a first aspect, the present disclosure relates to a computer-implemented method comprising the steps:
(a) providing a pre-trained conditional generative model, wherein the pre-trained conditional generative model was trained to reconstruct a plurality of reference representations of an examination area of a plurality of examination objects,
(b) providing a native representation of the examination area of a new examination object, wherein the native representation represents the examination area of the new examination object without contrast agent,
(c) providing a contrast-enhanced representation of the examination area of the new examination object, wherein the contrast-enhanced representation represents the examination area of the new examination object after administration of an amount of a contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) one or more times,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

In another aspect, the present disclosure provides a computer system comprising:
a processing unit; and
a memory storing an application program configured to perform, when executed by the processing unit, an operation, the operation comprising:
   (a) providing a pre-trained conditional generative model, wherein the pre-trained conditional generative model was trained to reconstruct a plurality of reference representations of an examination area of a plurality of examination objects,
   (b) providing a native representation of the examination area of a new examination object, wherein the native representation represents the examination area of the new examination object without contrast agent,
   (c) providing a contrast-enhanced representation of the examination area of the new examination object, wherein the contrast-enhanced representation represents the examination area of the new examination object after administration of an amount of a contrast agent,
   (d) generating one or more embeddings at least partially based on the native representation and/or the contrast-enhanced representation,
   (e) providing starting data,
   (f) generating a synthetic representation of the examination area of the new examination object based on the starting data using the pre-trained conditional generative model and using the one or more embeddings as condition,
   (g) generating a transformed synthetic representation based on the synthetic representation, wherein the transformed synthetic representation is a contrast-enhanced representation of the examination area of the new examination object in which the contrast-enhancement is reduced compared to the synthetic representation,
   (h) quantifying deviations between the transformed synthetic representation and the contrast-enhanced representation of the examination area of the new examination object,
   (i) reducing the deviations by modifying the starting data and/or the one or more embeddings and/or parameters of the pre-trained conditional generative model,
   (j) repeating steps (f) to (i) one or more times,
   (k) outputting and/or storing the synthetic representation of the examination area of the new examination object and/or a synthetic image of the examination area of the new examination object generated therefrom, and/or transmitting the synthetic representation and/or the synthetic image to a separate computer system.

In another aspect, the present disclosure provides a non-transitory computer readable storage medium having stored thereon software instructions that, when executed by a processing unit of a computer system, cause the computer system to perform the following steps:
(a) providing a pre-trained conditional generative model, wherein the pre-trained conditional generative model was trained to reconstruct a plurality of reference representations of an examination area of a plurality of examination objects,
(b) providing a native representation of the examination area of a new examination object, wherein the native representation represents the examination area of the new examination object without contrast agent,
(c) providing a contrast-enhanced representation of the examination area of the new examination object, wherein the contrast-enhanced representation represents the examination area of the new examination object after administration of an amount of a contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) one or more times,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

In another aspect, the present disclosure relates to a use of a contrast agent in an examination of an examination area of a new examination object, the examination comprising:
(a) providing a pre-trained conditional generative model, wherein the pre-trained conditional generative model was trained to reconstruct a plurality of reference representations of an examination area of a plurality of examination objects,
(b) generating a native representation of the examination area of the new examination object, wherein the native representation represents the examination area of the new examination object without contrast agent,
(c) generating a contrast-enhanced representation of the examination area of the new examination object, wherein the contrast-enhanced representation represents the examination area of the new examination object after administration of an amount of the contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) one or more times,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

**In** another aspect, the present disclosure relates to a contrast agent for use in an examination of an examination area of a new examination object, the examination comprising:
(a) providing a pre-trained conditional generative model, wherein the pre-trained conditional generative model was trained to reconstruct a plurality of reference representations of an examination area of a plurality of examination objects,
(b) generating a native representation of the examination area of the new examination object, wherein the native representation represents the examination area of the new examination object without contrast agent,
(c) generating a contrast-enhanced representation of the examination area of the new examination object, wherein the contrast-enhanced representation represents the examination area of the new examination object after administration of an amount of the contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) one or more times,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

In another aspect, the present disclosure provides a kit comprising a contrast agent and computer-readable program code that, when executed by a processing unit of a computer system, cause the computer system to execute the following steps:
(a) providing a pre-trained conditional generative model, wherein the pre-trained conditional generative model was trained to reconstruct a plurality of reference representations of an examination area of a plurality of examination objects,
(b) providing a native representation of the examination area of a new examination object, wherein the native representation represents the examination area of the new examination object without contrast agent,
(c) providing a contrast-enhanced representation of the examination area of the new examination object, wherein the contrast-enhanced representation represents the examination area of the new examination object after administration of an amount of the contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) one or more times,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

### BRIEF DESCRIPTION OF THE DRAWINGS

Fig. 1 shows schematically by way of example the pre-training of a conditional generative model.
Fig. 2 shows schematically by way of example the use of a pre-trained conditional generative model to generate a synthetic representation of the examination area of a new examination object.
Fig. 3 shows schematically an example of generating a transformed synthetic representation.
Fig. 4 shows schematically another example of using the pre-trained conditional generative model to generate a synthetic representation of the examination area of a new examination object.
Fig. 5 shows a schematic example of generating an embedding based on a representation of an examination area of an examination object.
Fig. 6 shows a schematic example of generating several embeddings using an encoder and combining the embeddings into a single embedding.
Fig. 7 illustrates a computer system according to some example implementations of the present disclosure in more detail.
Fig. 8 schematically shows an embodiment of the computer-implemented method of the present disclosure in the form of a flow chart.

### DETAILED DESCRIPTION

Various example embodiments will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides means for generating a synthetic representation of an examination area of an examination object.

In an embodiment of the present disclosure, the "examination object" is a living being.

In an embodiment of the present disclosure, the "examination object" is a mammal.

In an embodiment of the present disclosure, the "examination object" is a human.

The "examination area" is a part of the examination object, for example an organ or part of an organ or a plurality of organs or another part of the examination object.

For example, the examination area may be a liver, kidney, heart, lung, brain, stomach, bladder, prostate, intestine, pancreas, thyroid, breast, uterus, or a part of said parts or another part of the body of a mammal (for example a human).

In an embodiment, the examination area includes a liver or part of a liver or the examination area is a liver or part of a liver of a mammal, e.g. a human.

In a further embodiment, the examination area includes a brain or part of a brain or the examination area is a brain or part of a brain of a mammal, e.g. a human.

In a further embodiment, the examination area includes a heart or part of a heart or the examination area is a heart or part of a heart of a mammal, e.g. a human.

In a further embodiment, the examination area includes a thorax or part of a thorax or the examination area is a thorax or part of a thorax of a mammal, e.g. a human.

In a further embodiment, the examination area includes a stomach or part of a stomach or the examination area is a stomach or part of a stomach of a mammal, e.g. a human.

In a further embodiment, the examination area includes a pancreas or part of a pancreas or the examination area is a pancreas or part of a pancreas of a mammal, e.g. a human.

In a further embodiment, the examination area includes a kidney or part of a kidney or the examination area is a kidney or part of a kidney of a mammal, e.g. a human.

In a further embodiment, the examination area includes one or both lungs or part of a lung of a mammal, e.g. a human.

In a further embodiment, the examination area includes a breast or part of a breast or the examination area is a breast or part of a breast of a female mammal, e.g. a female human.

In a further embodiment, the examination area includes a prostate or part of a prostate or the examination area is a prostate or part of a prostate of a male mammal, e.g. a male human.

The term "synthetic" means that the synthetic representation is not the (direct) result of a physical measurement on a real object under examination, but that the synthetic representation has been generated by a conditional generative model. A synonym for the term "synthetic" is the term "artificial". A synthetic representation may however be based on one or more measured representations, i.e., the conditional generative model may be able to generate the synthetic representation based on one or more measured representations (and/or other/further data).

The synthetic representation is generated based on a native representation of the examination area of the examination object and a contrast-enhanced representation of the examination area of the examination object. The generation of the synthetic representation may be based on further data.

The native representation represents the examination area of the examination object without contrast agent.

The contrast-enhanced representation represents the examination area of the examination object after administration of a first amount of a contrast agent.

"Contrast agents" are substances or mixtures of substances that improve the depiction of structures and functions of the body in radiological examinations.

In computed tomography, iodine-containing solutions are usually used as contrast agents. In magnetic resonance imaging (MRI), superparamagnetic substances (for example iron oxide nanoparticles, superparamagnetic iron-platinum particles (SIPPs)) or paramagnetic substances (for example gadolinium chelates, manganese chelates, hafnium chelates) are usually used as contrast agents. In the case of sonography, liquids containing gas-filled microbubbles are usually administered intravenously. In positron emission tomography (PET) radiotracers are used as contrast agents. Contrast in PET images is caused by the differential uptake of the radiotracer in different tissues or organs. A radiotracer is a radioactive substance that is injected into the examination object. The radiotracer emits positrons. When a positron collides with an electron within the examination region of the examination object, both particles are annihilated, producing two gamma rays that are emitted in opposite directions. These gamma rays are then detected by a PET scanner, allowing the creation of detailed images of the body's internal functioning.

Examples of contrast agents can be found in the literature (see for example A.S.L. Jascinth et al.: Contrast Agents in computed tomography: A Review, Journal of Applied Dental and Medical Sciences, 2016, vol. 2, issue 2, 143-149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M.R. Nouh et al.: Radiographic and magnetic resonances contrast agents: Essentials and tips for safe practices, World J Radiol. 2017 Sep. 28; 9(9): 339-349; L.C. Abonyi et al.: Intravascular Contrast Media in Radiography: Historical Development & Review of Risk Factors for Adverse Reactions, South American Journal of Clinical Research, 2016, vol. 3, issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362; J. Trotter et al.: Positron Emission Tomography (PET)/Computed Tomography (CT) Imaging in Radiation Therapy Treatment Planning: A Review of PET Imaging Tracers and Methods to Incorporate PET/CT, Advances in Radiation Oncology (2023) 8, 101212).

The synthetic representation represents the examination area of the examination object after administration of a second amount of the contrast agent. The second amount is higher than the first amount.

In an embodiment of the present disclosure, the first amount is equal to or less than the standard amount. The standard amount is the amount recommended by the manufacturer and/or distributor of the contrast agent and/or the amount approved by a regulatory authority and/or the amount listed in a package insert for the contrast agent.

For example, the standard amount of Primovist^{®} is 0.025 mmol Gd-EOB-DTPA disodium/kg body weight.

In an embodiment of the present disclosure, the second amount is equal to or greater than the standard amount.

In an embodiment of the present disclosure, the first amount is less than the standard amount, and the second amount is equal to the standard amount.

In an embodiment of the present disclosure, the first amount is equal to or less than the standard amount, and the second amount is greater than the standard amount.

In an embodiment of the present disclosure, the first amount is equal to the standard amount, and the second amount is greater than the standard amount.

In an embodiment of the present disclosure, the first amount is less than the standard amount, and the second amount is greater than the standard amount.

In an embodiment of the present disclosure, the contrast agent is an MRI contrast agent. In an embodiment of the present disclosure, the contrast agent is an extracellular MRI contrast agent. In an embodiment of the present disclosure, the contrast agent is an intracellular MRI contrast agent. In an embodiment of the present disclosure, the contrast agent is a hepatobiliary MRI contrast agent.

A hepatobiliary contrast agent has the characteristic features of being specifically taken up by liver cells (hepatocytes), accumulating in the functional tissue (parenchyma) and enhancing contrast in healthy liver tissue. An example of a hepatobiliary contrast agent is the disodium salt of gadoxetic acid (Gd-EOB-DTPA disodium), which is described in US Patent No. 6 039 931A and is commercially available under the trade names Primovist^{®} and Eovist^{®}. Further hepatobiliary contrast agents are described inter alia in WO2022/194777.

In an embodiment of the present disclosure, the contrast agent is an agent that includes gadolinium(III) 2-[4,7,10-tris(carboxymethyl)-1,4,7,10-tetrazacyclododec-1-yl]acetic acid (also referred to as gadolinium-DOTA or gadoteric acid).

In a further embodiment, the contrast agent is an agent that includes gadolinium(III) ethoxybenzyldiethylenetriaminepentaacetic acid (Gd-EOB-DTPA); preferably, the contrast agent includes the disodium salt of gadolinium(III) ethoxybenzyldiethylenetriaminepentaacetic acid (also referred to as gadoxetic acid).

In an embodiment of the present disclosure, the contrast agent is an agent that includes gadolinium(III) 2-[3,9-bis[1-carboxylato-4-(2,3-dihydroxypropylaniino)-4-oxobutyl]-3,6,9,15-tetrazabicyclo[9.3.1]pentadeca-1(15),11,13-trien-6-yl]-5-(2,3-dihydroxypropylamino)-5-oxopentanoate (also referred to as gadopiclenol) (see for example WO2007/042504 and WO2020/030618 and/or WO2022/013454).

In an embodiment of the present disclosure, the contrast agent is an agent that includes dihydrogen [(±)-4-carboxy-5,8,11-tris(carboxymethyl)-1-phenyl-2-oxa-5,8,11-triazatridecan-13-oato(5-)]gadolinate(2-) (also referred to as gadobenic acid).

In one embodiment of the present disclosure, the contrast agent is an agent that includes tetragadolinium [4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetate (also referred to as gadoquatrane) (see for example J. Lohrke et al.: Preclinical Profile of Gadoquatrane: A Novel Tetrameric, Macrocyclic High Relaxivity Gadolinium-Based Contrast Agent. Invest Radiol., 2022, 1, 57(10): 629-638; WO2016193190).

In an embodiment of the present disclosure, the contrast agent is an agent that includes a Gd³⁺ complex of a compound of the formula (I) 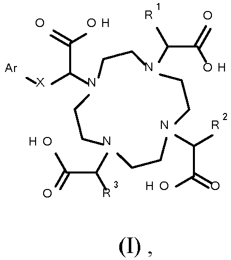 where
Ar is a group selected from 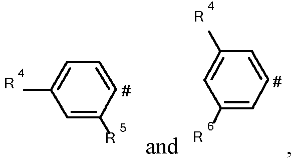
where # is the linkage to X,
X is a group selected from
CH₂, (CH₂)₂, (CH₂)₃, (CH₂)₄ and *-(CH₂)₂-O-CH₂-^{#} ,
where * is the linkage to Ar and ^{#} is the linkage to the acetic acid residue,
R¹, R² and R³ are each independently a hydrogen atom or a group selected from C₁-C₃ alkyl, -CH₂OH, -(CH₂)₂OH and -CH₂OCH₃,
R⁴ is a group selected from C₂-C₄ alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ is a hydrogen atom,
   and
R⁶ is a hydrogen atom,
or a stereoisomer, tautomer, hydrate, solvate or salt thereof, or a mixture thereof.

In an embodiment of the present disclosure, the contrast agent is an agent that includes a Gd³⁺ complex of a compound of the formula (II) 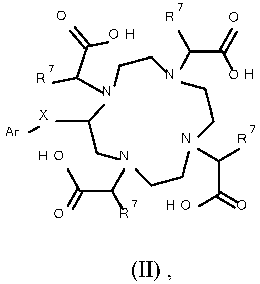 where
Ar is a group selected from 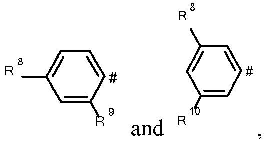
where # is the linkage to X,
X is a group selected from CH₂, (CH₂)₂, (CH₂)₃, (CH₂)₄ and *-(CH₂)₂-O-CH₂-^{#}, where * is the linkage to Ar and ^{#} is the linkage to the acetic acid residue,
R⁷ is a hydrogen atom or a group selected from C₁-C₃ alkyl, -CH₂OH, -(CH₂)₂OH and -CH₂OCH₃;
R⁸ is a group selected from
C₂-C₄ alkoxy, (H₃C-CH₂O)-(CH₂)₂-O-, (H₃C-CH₂O)-(CH₂)₂-O-(CH₂)₂-O- and
(H₃C-CH₂O)-(CH₂)₂-O-(CH₂)₂-O-(CH₂)₂-O-;
R⁹ and R¹⁰ independently represent a hydrogen atom;
or a stereoisomer, tautomer, hydrate, solvate or salt thereof, or a mixture thereof.

The term "C₁-C₃ alkyl" denotes a linear or branched, saturated monovalent hydrocarbon group having 1, 2 or 3 carbon atoms, for example methyl, ethyl, n-propyl or isopropyl. The term "C₂-C₄ alkyl" denotes a linear or branched, saturated monovalent hydrocarbon group having 2, 3 or 4 carbon atoms.

The term "C₂-C₄" refers to a linear or branched, saturated monovalent group of the formula (C₂-C₄ alkyl)-O-, in which the term "C₂-C₄ alkyl" is as defined above, for example a methoxy, ethoxy, n-propoxy or isopropoxy group.

In an embodiment of the present disclosure, the contrast agent is an agent that includes gadolinium 2,2',2"-(10-{1-carboxy-2-[2-(4-ethoxyphenyl)ethoxy]ethyl}-1,4,7,10-tetraazacyclododecane-1,4,7-triyl)triacetate (see for example WO2022/194777, example 1).

In an embodiment of the present disclosure, the contrast agent is an agent that includes gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate (see for example WO2022/194777, example 2).

In an embodiment of the present disclosure, the contrast agent is an agent that includes gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate (see for example WO2022/194777, example 4).

In an embodiment of the present disclosure, the contrast agent is an agent that includes gadolinium (2S,2'S,2"S)-2,2'2"-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate (see for example WO2022/194777, example 15).

In an embodiment of the present disclosure, the contrast agent is an agent that includes gadolinium 2,2',2"-{10-[(1S)-4-(4-butoxyphenyl)-1-carboxybutyl]-1,4,7,10-tetraazacyclododecane-1,4,7-triyl}triacetate (see for example WO2022/194777, example 31).

In an embodiment of the present disclosure, the contrast agent is an agent that includes gadolinium - 2,2',2"-{(2S)-10-(carboxymethyl)-2-[4-(2-ethoxyethoxy)benzyl]-1,4,7,10-tetraazacyclododecane-1,4,7-triyl}triacetate.

In an embodiment of the present disclosure, the contrast agent is an agent that includes gadolinium 2,2',2"-[10-(carboxymethyl)-2-(4-ethoxybenzyl)-1,4,7,10-tetraazacyclododecane-1,4,7-triyl]triacetate.

In an embodiment of the present disclosure, the contrast agent is an agent that includes gadolinium(III) 5,8-bis(carboxylatomethyl)-2-[2-(methylamino)-2-oxoethyl]-10-oxo-2,5,8,11-tetraazadodecane-1-carboxylate hydrate (also referred to as gadodiamide).

In an embodiment of the present disclosure, the contrast agent is an agent that includes gadolinium(III) 2-[4-(2-hydroxypropyl)-7,10-bis(2-oxido-2-oxoethyl)-1,4,7,10-tetrazacyclododec-1-yl]acetate (also referred to as gadoteridol).

In an embodiment of the present disclosure, the contrast agent is an agent that includes gadolinium(III) 2,2',2"-(10-((2R,3S)-1,3,4-trihydroxybutan-2-yl)-1,4,7,10-tetraazacyclododecane-1,4,7-triyl)triacetate (also referred to as gadobutrol or Gd-DO3A-butrol).

Each representation (the synthetic representation, the native representation, and the contrast-enhanced representation) may be an image, i.e., a representation in real space; however, it may also be a representation in another space, such as a representation in frequency space or a representation in projection space.

In a representation in real space, also referred to in this disclosure as real-space representation, the examination area is normally represented by a number of image elements (for example pixels or voxels or doxels), which may for example be in a raster arrangement in which each image element represents a part of the examination area, wherein each image element may be assigned a colour value or grey value. The colour value or grey value represents a signal intensity, for example the attenuation of X-rays in case of X-ray images. A format widely used in radiology for storing and processing representations in real space is the DICOM format. DICOM (Digital Imaging and Communications in Medicine) is an open standard for storing and exchanging information in medical image data management.

In a representation in frequency space, also referred to in this description as frequency-space representation, the examination area is represented by a superposition of fundamental vibrations. For example, the examination area may be represented by a sum of sine and cosine functions having different amplitudes, frequencies, and phases. The amplitudes and phases may be plotted as a function of the frequencies, for example, in a two- or three-dimensional plot. Normally, the lowest frequency (origin) is placed in the centre. The further away from this centre, the higher the frequencies. Each frequency can be assigned an amplitude representing the frequency in the frequency-space representation and a phase indicating the extent to which the respective vibration is shifted towards a sine or cosine vibration. The k-space data produced by magnetic resonance imaging (MRI) is an example of a representation in frequency space.

A representation in real space can for example be converted (transformed) by a Fourier transform into a representation in frequency space. Conversely, a representation in frequency space can for example be converted (transformed) by an inverse Fourier transform into a representation in real space.

Details about real-space depictions and frequency-space depictions and their respective interconversion are described in numerous publications, see for example https://see.stanford.edu/materials/lsoftaee261/book-fall-07.pdf.

The representation of an examination area in the projection space can, for example, be the result of a computer tomographic examination prior to image reconstruction. In other words: the raw data obtained in the computed tomography examination can be understood as a projection-space representation. In computed tomography, the intensity or attenuation of X-ray radiation as it passes through the examination object is measured. From this, projection values can be calculated. In a second step, the object information encoded by the projection is transformed into an image (real-space depiction) through a computer-aided reconstruction. The reconstruction can be effected with the Radon transform. The Radon transform describes the link between the unknown examination object and its associated projections.

Details about the transformation of projection data into a real-space representation are described in numerous publications, see for example K. Catch: The Radon Transformation and Its Application in Tomography, Journal of Physics Conference Series 1903(1):012066.

For the sake of simplicity, the aspects of the present disclosure are described in some parts of this disclosure using the example of images as representations; however, this should not be construed as limiting the disclosure. A person skilled in the art of image processing will know how the teachings of the present disclosure can be applied to representations other than images.

The term "image" as used herein means a data structure that represents a spatial distribution of a physical signal. The spatial distribution may be of any dimension, for example 2D, 3D, 4D or any higher dimension. The spatial distribution may be of any shape, for example forming a grid and thereby defining pixels or voxels, the grid being possibly irregular or regular. The physical signal may be any signal, for example proton density, tissue echogenicity, tissue radiolucency, measurements related to the blood flow, information of rotating hydrogen nuclei in a magnetic field, color, level of gray, depth, surface or volume occupancy, such that the image may be a 2D or 3D RGB/grayscale/depth image, or a 3D surface/volume occupancy model. An image is usually composed of discrete image elements (e.g., pixels for 2D images, voxels for 3D images, doxels for 4D images).

Each representation (the synthetic representation, the native representation, and the contrast-enhanced representation) is a radiological representation. "Radiology" is the branch of medicine concerned with the application of electromagnetic radiation and mechanical waves (including, for example, ultrasound diagnostics) for diagnostic, therapeutic and/or scientific purposes. In addition to X-rays, other ionizing radiation such as gamma rays or electrons are also used. Since a primary purpose is imaging, other imaging procedures such as sonography and magnetic resonance imaging (MRI) are also included in radiology, although no ionizing radiation is used in these procedures. Thus, the term "radiology" as used in the present disclosure includes, in particular, the following examination procedures: computed tomography, magnetic resonance imaging, sonography, positron emission tomography (PET).

Each radiological representation can be, e.g., a 2D or 3D CT scan or MRI scan.

The synthetic representation is generated using a pre-trained conditional generative model.

A "generative model" is a type of machine learning model that is designed to learn and generate new data that resembles the training data it was trained on. Generative models capture the underlying distribution of the training data and can generate samples from that distribution.

A "conditional generative model" is a type of generative model that generates data (in this case, synthetic representations) given certain conditions or constraints. Conditional generative models take additional input in the form of a condition that guides the process of generating the synthetic representation. In general, this condition can be anything that provides some sort of context for the generation process, such as a class label, a text description, an image, or any other piece of information.

In an embodiment of the present disclosure, the pre-trained conditional generative model is or comprises a diffusion model, or at least the denoising model of a diffusion model.

Diffusion models focus on modeling the step-by-step evolution of a data distribution from a "simple" starting point to a "more complex" distribution. The underlying concept of diffusion models is to transform a simple and easily sampleable distribution, typically a Gaussian distribution, into a more complex data distribution of interest. This transformation is achieved through a series of invertible operations. Once the model learns the transformation process, it can generate new samples by starting from a point in the simple distribution and gradually "diffusing" it to the desired complex data distribution.

When (pre-)trained, a diffusion model usually comprises a noising model and a denoising model.

The noising model usually comprises a plurality of noising stages. The noising model is configured to receive input data (e.g., an image) and produce noisy data in response to receipt of the input data. The noising model introduces noise to the input data to obfuscate the input data after a number of stages, or "timesteps" *T.* The noising model can be or can include a finite number of steps *T* or an infinite number of steps (*T*→ ∞). The noising model may have the same weights/architectures for all timesteps or different weights/architectures for each timestep. The number of timesteps can be global (i.e., timesteps are the same for all pixels of an image) or local (e.g., each pixel in an image might have a different timestep).

The denoising model is configured to reconstruct the input data from noise data. The denoising model is configured to produce samples matching the input data after a number of stages.

For example, the diffusion model may include Markov chains at the noising model and/or denoising model. The diffusion models may be implemented in discrete time, e.g., where each layer corresponds to a timestep. The diffusion model may also be implemented in arbitrarily deep (e.g., continuous) time.

Diffusion models can be conceptually similar to a variational autoencoder (VAE) whose structure and loss function provides for efficient training of arbitrarily deep (e.g., infinitely deep) models. The diffusion model can be trained using variational inference, for example.

The diffusion model can be a Latent Diffusion Model (LDM). In such a model, the diffusion approach in the case of an image is not performed in real space (e.g., pixel space or voxel space or doxel space, as the case may be), but in so-called latent space based on a representation of the image, usually a compressed representation (see, e.g., R. Rombach et al.: High-Resolution Image Synthesis with Latent Diffusion Models, arXiv:2112.10752v2).

The diffusion model may be a Denoising Diffusion Probabilistic Model (DDPM). DDPMs are a class of generative models that work by iteratively adding noise to input data (e.g., an image or a compressed representation) and then learning to denoise from the noisy signal to generate new samples (see, e.g., J. Ho et al.: Denoising Diffusion Probabilistic Models, arXiv:2006.11239v2).

The diffusion model may be a Score-based Generative Model (SGM). In SGMs the data is perturbed with random Gaussian noise of various magnitudes. With the gradient of log probability density as score function, samples are generated towards decreasing noise levels and the model is trained by estimating the score functions for noisy data distribution (see, e.g., Y. Song et al.: Score-Based Generative Modeling through Stochastic Differential Equations, arXiv:2011.13456v2).

The diffusion model may be a Denoising Diffusion Implicit Model (DDIM) (see, e.g.: J. Song et al.: Denoising Diffusion Implicit Models, arXiv:2010.02502v4). A critical drawback of DDPMs is that they require many iterations to produce a high-quality sample. For DDPMs, this is because the generative process (from noise to data) approximates the reverse of the forward diffusion process (from data to noise), which could have thousands of steps; iterating over all the steps is required to produce a single sample. DDIMs are implicit probabilistic models that are closely related to DDPMs, in the sense that they are trained with the same objective function. DDIMs allow for much faster sampling while keeping an equivalent training objective. They do this by estimating the addition of multiple Markov chain steps and adding them all at once. DDIMs construct a class of non-Markovian diffusion processes which makes sampling from reverse process much faster. This modification in the forward process preserves the goal of DDPM and allows for deterministically encoding an image to the noise map.

Unlike DDPMs, DDIMs enable control over image synthesis owing to the latent space flexibility (attribute manipulation) (see, e.g., K. Preechakul *et al*.: *Diffusion autoencoders: Toward a meaningful and decodable representation,* arXiv:2111.15640v3). With DDIM, it is possible to run the generative process backward deterministically to obtain the noise map x*_{T}*, which represents the latent variable or encoding of a given image x₀. In this context, DDIM can be thought of as an image decoder that decodes the latent code x*_{T}* back to the input image. This process can yield a very accurate reconstruction; however, x*_{T}* still does not contain high-level semantics as would be expected from a meaningful representation.

In an embodiment of the present disclosure, the conditional generative model is or comprises a conditional diffusion model.

In a conditional diffusion model, a condition is used to denoise latent data and reconstruct the input data (see, e.g., P. Dhariwal, A. Nichol: *"Diffusion models beat GANs on image synthesis,"* arXiv:2105.05233v4). One benefit of conditioning the diffusion model with information-rich representations is a more efficient denoising process.

In general, such a condition can be based on a text (e.g., text-to-image), on an image, on audio data, or on other information.

The conditional generative model is a pre-trained machine learning model.

The term "pre-trained" refers to a model that has been trained on a training dataset in advance and is made available for use. Pre-training often involves training a model on a task or dataset that is different from the specific task for which the model will be used later. The pre-training process involves exposing the model to a vast amount of data and allowing it to learn general patterns and representations from that data. This enables the model to capture common features and structures that are useful across various related tasks. The model is typically trained using unsupervised or self-supervised learning methods, where the target data are generated automatically or do not require human intervention. Once the pre-training phase is complete, the model's weights and parameters can be saved and made available.

The pre-trained conditional generative model of the present disclosure was trained to reconstruct a plurality of reference representations of the examination area of a plurality of examination objects.

The term "plurality" means more than ten, e.g., more than a hundred or even more than a thousand.

The training data is generated based on a plurality of data sets from a plurality of examination objects.

The pre-training includes the following steps, which are explained in more detail below:
- providing a training data set, the training data set comprising a plurality of reference representations of the examination area of a plurality of examination objects,
- providing a conditional generative model, wherein the conditional generative model is configured to reconstruct each reference representation of the multitude of reference representations,
- pre-training the conditional generative model on the training data set,
- storing the pre-trained conditional generative model.

The term "reference" is used in this description to distinguish the phase of pre-training the conditional generative model from the phase of using the pre-trained model for generating a synthetic representation. The term "reference" otherwise has no limitation on meaning. A "reference representation" is a representation of the examination area of an examination object which is used to pre-train the conditional generative model. If the pre-trained conditional generative model is used to generate a synthetic representation for an examination object, the examination object is also referred to as a "new" examination object in this disclosure. Again, the term "new" is used only to distinguish the pre-training phase from the phase of using the pre-trained conditional generative model for generating synthetic representations.

In a first step, a training data set is provided. The training data set comprises a plurality of reference representations of the examination area of a plurality of examination objects.

Each examination object of the plurality of examination objects is a living being such as an animal, e.g. a mammal, and/or a human. In an embodiment of the present disclosure, each examination object is a human.

The examination area is a part of the examination object, e.g., a liver, kidney, heart, lung, brain, stomach, bladder, prostate, intestine, eye, breast, thyroid, pancreas, uterus, or a part of said parts or another part of the examination object.

The examination area is usually the same for all examination objects.

Each representation of the examination area of each examination object is usually a radiologic representation. The radiologic representation may by a representation with or without contrast. In other words, the training data set comprises a plurality of reference representations of the examination area of a plurality of examination objects, the reference representations comprising native and/or contrast-enhanced radiologic representations.

The amount of contrast agent administered to an examination object may be the same for some or all contrast-enhanced representations or may vary for all.

The training data may comprise pairs of reference representations for one or more examination objects, such a pair comprising one reference representation being a native reference representation representing the examination area of the examination object without contrast agent, and the other reference representation representing the examination area after administration of a contrast agent.

The training data may comprise triplets of reference representations for one or more examination objects, such triplet comprising a first reference representation being a native reference representation representing the examination area of the examination object without contrast agent, a second reference representation representing the examination area after administration of a first amount of a contrast agent, and a third reference representation representing the examination area after administration of a second amount of a contrast agent.

Each reference representation can be a representation in real space (image space, spatial domain), a representation in frequency space (frequency domain), a representation in the projection space or a representation in another space, as described above.

An embedding is generated for each reference representation. It is possible that more than one embedding is generated for one or more or all reference representations. An embedding is generated based on the respective reference representation. It is possible that one or more embeddings are generated based on more than one reference representation of an examination object.

An "embedding" is a numerical representation of a reference representation that captures the salient features of the reference representation. The embedding can be a vector or a matrix or a tensor or another arrangement of numbers.

An embedding usually captures the meaning or semantics of the reference representation. It aims to encode the high-level information and concepts present in the reference representation, allowing machines to understand and reason about the content of the reference representation.

Embeddings are generated with the help of an encoder. The encoder can be part of the conditional generative model or a separate unit.

An embedding can be obtained, for example, by passing the reference representation through a pre-trained machine learning model and then extracting the output of one layer of the machine learning model. The machine learning model for generating embeddings can be or comprise a (pre-trained) convolutional neural network, for example.

Convolutional neural networks (CNNs) are frequently used for generating image embeddings. These artificial neural networks consist of multiple layers that progressively extract features at different levels of abstraction, capturing both low-level details and higher-level semantic concepts. The CNN can be part of a classifier or autoencoder, for example.

In an embodiment of the present disclosure, the embeddings are generated with an encoder of an optionally pre-trained autoencoder.

An "autoencoder" is a type of neural network architecture that is primarily used for unsupervised learning and dimensionality reduction. It may be designed to learn a compressed representation of the input data and then reconstruct the original data from this compressed representation (the embedding). An autoencoder usually comprises two main components: an encoder and a decoder. The encoder takes the input data and maps it to a lower-dimensional latent space representation, also known as the embedding. The decoder then takes this embedding and reconstructs the original input data from it. The objective of an autoencoder is to minimize the reconstruction error, which encourages the model to learn a compressed representation that captures the most salient features of the input data.

An autoencoder is often implemented as an artificial neural network that comprises a convolutional neural network (CNN) to extract features from, e.g., images as input data. An example of such an autoencoder is the U-Net (see, e.g., O. Ronneberger et al.: U-net: Convolutional networks for biomedical image segmentation, International Conference on Medical image computing and computer-assisted intervention, 234-241, Springer, 2015, DOI: 10.1007/978-3-319-24574-4_28). Further examples of autoencoders are sparse autoencoders, denoising autoencoders, variational autoencoders (VAEs), and generative adversarial networks (GANs). The autoencoder can be (pre-)trained based on (e.g., non-annotated) reference representations.

Autoencoders can be (pre-)trained using a self-supervised learning approach, meaning they do not require labeled data for training.

In another embodiment of the present disclosure, the embeddings are generated with the help of a pre-trained vision transformer.

Transformers are widely used for various natural language processing tasks, including machine translation, text summarization, sentiment analysis, and more.

At the core of the transformer model is the transformer architecture, which relies heavily on attention mechanisms to process sequential data efficiently. Unlike traditional recurrent neural networks (RNNs) or convolutional neural networks (CNNs), transformers do not employ recurrent or convolutional operations. Instead, they use attention mechanisms to capture contextual relationships between, e.g., words or tokens in a sequence.

The transformer architecture consists of two main components: the encoder and the decoder. The encoder processes the input sequence, modeling its contextual relationships, while the decoder generates the output sequence based on the encoded information. Both the encoder and decoder are usually composed of multiple layers of attention mechanisms and feed-forward neural networks. The attention mechanism allows the model to focus on different parts of the input sequence while considering the dependencies between tokens.

Transformers have significantly contributed to advancements in machine learning, particularly in natural language processing tasks. Their ability to capture contextual information efficiently has resulted in state-of-the-art performance on various benchmarks and has paved the way for numerous applications in the field (see, e.g., T. Lin et al.: A survey of transformers, AI Open, Volume 3, 2022, Pages 111-132).

Transformers have been applied to images (vision transformer). In a vision transformer, the input image is divided into a sequence of patches, which are then flattened and fed into a series of transformer layers. These transformer layers comprise attention modules and feed-forward neural networks. The attention mechanism allows the model to capture the relationships between different patches and learn global context information, while the feed-forward networks enable non-linear transformations (see, e.g., S. Khan et al.: Transformers in Vision: A Survey, arXiv:2101.01169v5).

The key advantage of vision transformers is their ability to model long-range dependencies and capture global context, which is crucial for understanding complex visual patterns and relationships.

Like the autoencoder, the (vision) transformer is preferably pre-trained. The (vision) transformer may have been pre-trained in a supervised, self-supervised or unsupervised approach.

"Self-supervised learning" is a type of machine learning paradigm where a model is trained to learn from the data itself, without the need for human-labelled annotations. Instead of relying on external labels provided by humans, the model generates its own supervisory signals from the input data, making it a form of unsupervised learning.

The (vision) transformer may have been pre-trained in a DINO approach. DINO (self-DIstillation with NO labels) is a self-supervised learning method specifically designed to improve the performance of vision transformers in image classification tasks (see, e.g., M. Caron et al.: Emerging Properties in Self-Supervised Vision Transformers, arXiv:2104.14294v2).

DINO introduces a novel approach to self-supervised learning for vision transformers by leveraging two main components: clustering and distillation. Initially, the model is trained to cluster the augmented views of the input data. This clustering helps the model to discover semantically similar instances within the dataset. Then, a distillation process is performed, where the model learns to transfer knowledge from a teacher network to a student network. The teacher network provides soft targets, or guidance, to the student network, which helps improve the student's performance. By combining clustering and distillation, DINO enables the model to learn more robust and discriminative representations, leading to better generalization and performance on downstream tasks such as image classification.

In another embodiment of the present disclosure, the vision transformer is pre-trained using a DiNOv2 approach. DiNOv2 (DIscriminative NOise Contrastive Learning V2) is another self-supervised approach for training vision transformers (see, e.g., M. Oquab et al.: DINOv2: Learning Robust Visual Features without Supervision, arXiv:2304.07193v1).

In another embodiment of the present disclosure, the embeddings are embeddings generated with the help of an encoder of a pre-trained CLIP model.

CLIP (Contrastive Language-Image Pretraining) is a framework in the field of machine learning that combines natural language processing and computer vision to understand and generate multimodal representations of images and text. CLIP encodes text and image in same embedding space (see, e.g., A. Radford et al.: Learning Transferable Visual Models From Natural Language Supervision, arXiv:2103.00020v1).

CLIP is (pre-)trained in a self-supervised manner, where large-scale datasets of images and their associated text are used to learn joint representations. The model is trained to associate images and their textual descriptions by maximizing their similarity in the learned embedding space. This allows CLIP to understand and reason about images and text in a shared semantic space. The base model uses a ViT-L/14 transformer architecture as an image encoder and uses a masked self-attention transformer as a text encoder. These encoders are trained to maximize the similarity of (image, text) pairs via a contrastive loss.

The key innovation of CLIP is its ability to generalize across different domains and tasks. By training on a diverse range of image and text pairs, CLIP can perform a variety of tasks without task-specific fine-tuning. For example, CLIP can perform zero-shot image classification, where it can classify images into categories it has never seen during training, solely based on textual descriptions.

In a preferred embodiment, the image encoder of a pre-trained CLIP model is used which was pre-trained on medical images, such as BiomedCLIP (see, e.g., S. Zhang et al.: Large-Scale Domain-Specific Pretraining for Biomedical Vision-Language Processing, arXiv:2303.00915v1).

However, the models and training procedures listed here are only examples; the embeddings can also be generated in other ways.

It is also possible to generate more than one embedding of a reference representation (e.g., two, or three or four or any other number).

It is possible to generate different embeddings from a reference representation (e.g. using different embedding methods).

If multiple embeddings are available, these can be combined into a single embedding, e.g. through concatenation, average pooling, attention-weighted pooling and/or other combination methods.

Embeddings can be generated before pre-training the conditional generative model and then saved. Embeddings can also be generated during pre-training of the conditional generative model.

Generating an embedding of one or more reference representations usually comprises: inputting the one or more reference representations into an encoder, and receiving the embedding of the one or more reference representations as an output of the encoder.

Pre-training the conditional generative model usually involves the following steps:
For each data set:
- inputting the reference representation and the at least one embedding of the reference representation into the conditional generative model, wherein the at least one embedding of the reference representation is used as a condition in the generation of a reconstructed reference representation,
- receiving the reconstructed reference representation as an output of the conditional generative model,
- determining deviations between the reference representation and the reconstructed reference representation,
- reducing the deviations by modifying model parameters of the conditional generative model.

The pre-training of the conditional generative model can be ended when a stop criterion is met. Such a stop criterion can be for example: a predefined maximum number of training steps/cycles/epochs has been performed, deviations between output data and target data can no longer be reduced by modifying the model parameters, a predefined minimum of the loss function is reached, and/or an extreme value (e.g., maximum or minimum) of another performance value is reached.

The pre-trained conditional generative model can be saved, transferred to a separate computer system and/or used to generate a synthetic representation of the examination area of a new examination object.

The reference representations of the training data set can be or comprise annotated representations. In other words, for some or all reference representations, an annotation may be available.

The annotation may provide information about the nature of the reference representation (e.g., CT representation, MRI representation, microscopic image, and/or the like), what is represented by the representation (e.g., which examination area) and/or from which examination object the representation was obtained, and/or how the representation was created (e.g., which measurement protocol was used, whether a contrast agent was administered, which contrast agent was administered, and/or the amount of contrast agent administered, if applicable, which radiation dose was used, at which time the representation was generated (e.g., before or after administration of a contrast agent)), and/or other/further information about the representation and/or the content of the representation and/or the generation of the representation.

However, it is also possible that the reference representations of the training data set are or comprise non-annotated representations.

From each annotation (if available), a semantic representation may be generated. In case of text, such a semantic representation can be generated by a text encoder, e.g., a text encoder of a CLIP model (see, e.g., A. Radford et al.: Learning Transferable Visual Models From Natural Language Supervision, arXiv:2103.00020v1), or any other word embedding technique (see, e.g., S. Selva Birunda and R. Kanniga Devi: A Review on Word Embedding Techniques for Text Classification in Innovatove Data Communication Technologies and Application, Proceedings of ICIDCA 2020, Vol. 59, Springer, ISSN 2367-4512, 267-281; M. T. Pilehvar, J. Camacho-Collados: Embeddings in Natural Language Processing, Morgan&Claypool Publishers 2021, ISBN: 9781636390222).

If the annotation is not a text file, another encoder can be used to generate a semantic representation of the annotation, such as an image encoder, an encoder of a pre-trained autoencoder, and/or the like.

The semantic representation may be used as additional conditional input to the conditional generative model.

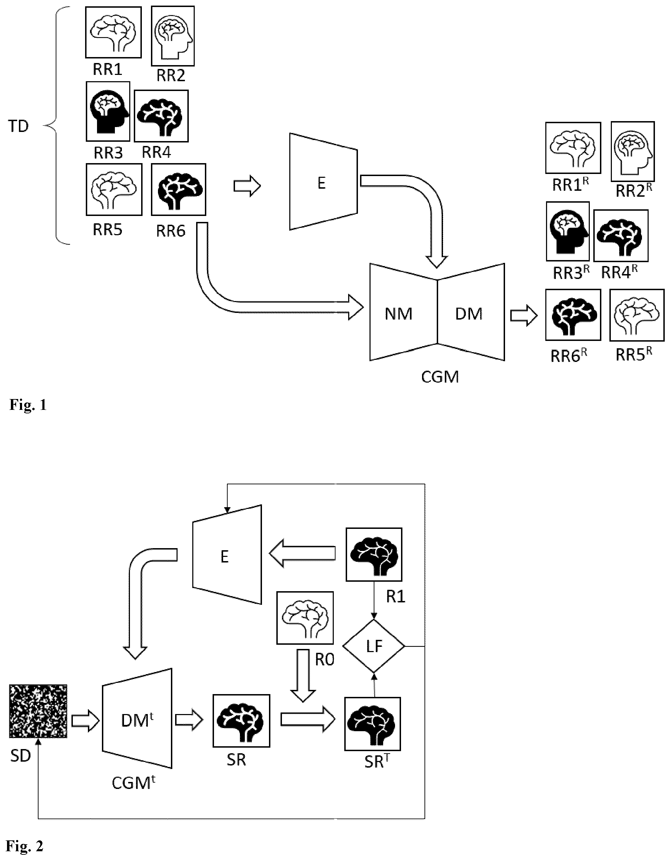

Fig. 1 shows schematically by way of example the pre-training of a conditional generative model.

In the example depicted in Fig. 1, the conditional generative model CGM is a diffusion model. The diffusion model comprises a noising model NM and a denoising model DM.

The conditional generative model CGM is pre-trained on training data TD.

The training data TD comprise a multitude of reference representations RR1, RR2, RR3, RR4, RR5, RR6 of an examination area of a multitude of examination objects.

In the example shown in Fig. 1, the examination area comprises the brain of a multitude of humans.

The conditional generative model CGM is configured and trained to gradually add noise to the reference representations RR1, RR2, RR3, RR4, RR5, RR6 and regenerate (reconstruct) the reference representations from the noise data (denoising).

An encoder E (e.g., an image encoder) is used to generate one or more embeddings for each reference representation RR1, RR2, RR3, RR4, RR5, RR6.

The one or more embeddings are used as conditions when reconstructing the reference representation.

In Fig. 1, the reconstructed reference representations are marked with the reference signs RR1^{R}, RR2^{R}, RR3^{R}, RR4^{R}, RR5^{R}, and RR6^{R}.

Of course, augmentation and masking techniques can be used in the reconstruction of the reference representations.

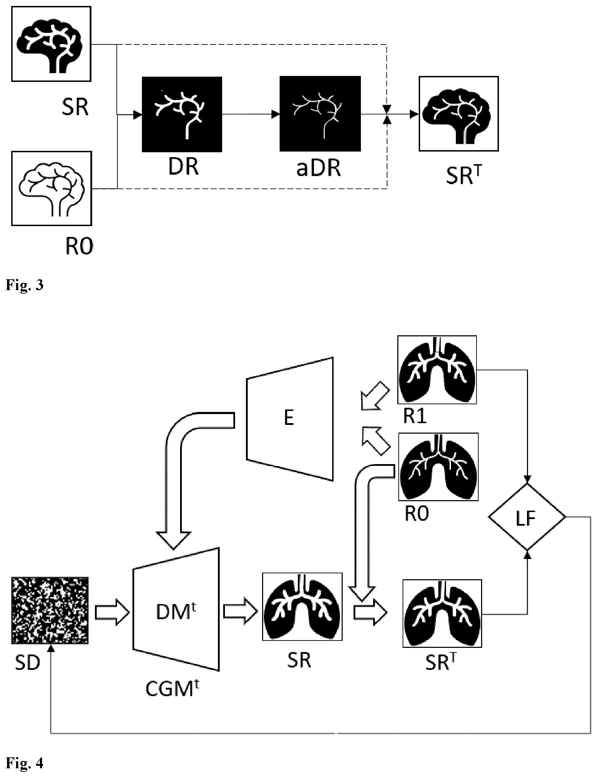

Once the conditional generative model CGM has been pre-trained, it can be used to generate a synthetic representation of the examination area of a new examination object. This is shown in Figs. 2 and 4.

In a first step, a native representation and a contrast-enhanced representation of the examination area of the new examination object is provided. The contrast-enhanced representation serves as target data.

The native representation and/or the contrast-enhanced representation are usually measured data, i.e., the result of a measurement on the new examination object. It is possible that the measured data is processed or transformed to generate the respective representation of the examination area of the new examination object. For example, the measured data may be k-space data that is subjected to an inverse Fourier transform to generate a real-space representation of the examination area of the new examination object (however, this does not mean that the representation of the examination area of the examination object cannot consist of k-space data).

One or more embeddings are generated based on the native and/or contrast-enhanced representation. The one or more embeddings are preferably generated with the same encoder that was used to generate the embeddings of the reference representations during training of the conditional generative model. In other words, everything that is disclosed in this disclosure for the embeddings of the reference representation also applies analogously to the one or more embeddings of the native representation and/or the contrast-enhanced representation.

Starting data is provided for the generation of the synthetic representation. The conditional generative model is configured to generate the synthetic representation based on the starting data, using the one or more embeddings as a condition. In the case of a diffusion model, for example, the starting data can be noise data. The noise data can be random Gaussian noise, for example.

The conditional generative model may be configured and pre-trained to gradually denoise the noise data and generate a synthetic representation of the examination area of the new examination object.

In a next step, the synthetic representation is transformed. The result of the transformation operation is a transformed synthetic representation of the examination area of the new examination object. In other words, generating a transformed synthetic representation may comprise: reducing the contrast-enhancement of the synthetic representation. Generating a transformed synthetic representation may comprise: reducing the contrast-enhancement of the synthetic representation, so that the contrast-enhancement of the transformed synthetic representation corresponds to the contrast-enhancement of the contrast-enhanced representation (target data). Generating a transformed synthetic representation may comprise: reducing the contrast-enhancement of the synthetic representation, so that the transformed synthetic representation represents the examination area of the new examination object after administration of the first amount of contrast agent.

The transformed synthetic representation is a contrast-enhanced representation of the examination area of the new examination object in which the contrast is reduced compared to the synthetic representation.

In other words, the transformation is intended to undo what the conditional generative model is supposed to achieve:
Based on a native representation representing the examination area of the new examination object without contrast agent and/or a contrast-enhanced representation representing the examination area of the new examination object after administration of a first amount of a contrast agent, the conditional generative model shall generate a synthetic contrast-enhanced representation representing the examination area after administration of a second amount of the contrast agent, wherein the second amount is larger than the first amount. By means of the transformation, a transformed synthetic representation is to be generated from the synthetic representation, which represents the examination area of the new examination object after administration of the first amount of the contrast agent. The transformed synthetic representation should therefore come as close as possible to the contrast-enhanced representation (target data).

In other words, the aim of the transformation is to convert the synthetic representation so that it corresponds to the (e.g., measured) contrast-enhanced representation of the examination area of the new examination object (target data).

The transformed synthetic representation is compared with the contrast-enhanced representation of the examination area of the new examination object (target data)
A loss function may be used to quantify deviations between the contrast-enhanced representation of the examination area of the new examination object and the transformed synthetic representation.

The deviations may be reduced in an optimization process by modifying the starting data and/or the one or mode embeddings and/or parameters of the pre-trained conditional generative model. A new synthetic representation is generated with the modified starting data and/or the modified embedding(s) and/or the modified parameters. This is then converted into a new transformed synthetic representation and deviations between the new transformed synthetic representation and the contrast-enhanced representation of the examination area of the new examination object is quantified. The procedure may be continued until a stop criterion is reached.

Such stop criteria can be for example: a predefined maximum number of steps has been performed, deviations between output data and target data can no longer be reduced by modifying the starting data and/or embedding(s) and/or model parameters, a predefined minimum of the loss function is reached, and/or an extreme value (e.g., maximum or minimum) of another performance value is reached.

Once the stop criterion has been reached, the last synthetic representation generated can be output (e.g., shown on a display and/or printed out on a printer), stored in a data memory and/or transferred to another computer system.

It is also possible that the last generated synthetic representation is first converted into another representation, which is then output, stored and/or transferred to a separate computer system. For example, it is possible for the last generated synthetic representation to be a representation in frequency space or projection space and for this to be converted into a real-space representation.

Modifications are usually made as part of an optimization process, e.g. a gradient descent optimization process.

Modifying the one or more embeddings may comprise modifying model parameters of the one or more encoders used to generate the one or more embeddings.

Modifying the one or more embeddings may include modifying the manner in which multiple (i.e., more than one) embeddings are combined into a single embedding.

Fig. 2 shows schematically by way of example the use of a pre-trained conditional generative model to generate a synthetic representation of the examination area of a new examination object. The pre-trained conditional generative model may have been trained as described with reference to Fig. 1.

In the example depicted in Fig. 2, the pre-trained conditional generative model CGM^{t} comprises a pre-trained denoising model DM^{t} of a diffusion model. The noising model NM shown in Fig. 1 is not required to generate synthetic representations. It may be discarded.

In a first step, a native representation R0 and a contrast-enhanced representation R1 of the examination area of a new examination object are provided. The native representation R0 and the contrast-enhanced representation R1 are radiologic representations, such as CT representations or MRI representations.

The conditional generative model CGM^{t} has been pre-trained to reconstruct reference representations of human brains (see Fig. 1), and the representations R0 and R1 also represent a human brain.

The native representation R0 represents the examination area of the new examination object without contrast agent. The contrast-enhanced representation R1 represents the examination area of the new examination object after administration of a first amount of a contrast agent.

The representation R1 serves as target data. One or more embeddings are generated from the representation R1 with the help of the encoder E, which are used as a condition for generating the synthetic representation SR.

The synthetic representation SR is generated based on the starting data SD. The denoising model DM^{t} of the pre-trained conditional generative model CGM^{t} is configured and pre-trained to generate the synthetic representation SR based on the starting data SD under the conditions specified by the one or more embeddings generated by the encoder E.

In a further step, the synthetic representation SR is converted into the transformed synthetic representation SR^{T}. In the example shown in Fig. 2, the native representation R0 is involved in the transformation.

The transformed synthetic representation SR^{T} is compared with the contrast-enhanced representation R1. Deviations between the transformed synthetic representation SR^{T} and contrast-enhanced representation R1 are quantified (e.g., by using the loss function LF) and the deviations are reduced by modifying the starting data SD and/or the one or more embeddings generated by the encoder E and/or parameters of the conditional generative model CGM^{t}.

The modified starting data and/or embedding(s) and/or modified parameters form the basis for a further optimization cycle, i.e. the steps described from the generation of the synthetic representation to the modification of the starting data and/or embedding(s) can be repeated once or several times. With each cycle, the deviations usually become smaller and the quality of the synthetic representation better.

Once a stop criterion has been reached, the last synthetic representation generated can be output (e.g., shown on a display and/or printed out on a printer), stored in a data memory and/or transferred to another computer system.

Such stop criteria can be for example: a predefined maximum number of steps has been performed, deviations between the transformed synthetic representation SR^{T} and the contrast-enhanced representation R1 can no longer be reduced by modifying the starting data SD and/or the one or more embeddings and/or the model parameters, a predefined minimum of the loss function is reached, and/or an extreme value (e.g., maximum or minimum) of another performance value is reached.

In the example shown in Fig. 2, one or more embeddings are generated by the encoder E only based on the representation R1. It is also possible that one or more embeddings are only generated based on the representation R0. It is possible that one or more embeddings are generated based on the representations R1 and R0. It is possible that one or more embeddings are generated based on the difference between the representation R0 and the representation R1 (e.g., R1-R0). It is possible that more than one encoder is used to generate embeddings. If several embeddings are generated, they can be combined into a single embedding as described herein.

As described, the synthetic representation generated by the conditional generative model is transformed and the transformed synthetic representation is compared with the target data in order to quantify deviations. The transformation of the synthetic representation is a process that reverses what the conditional generative model is intended to achieve.

In an embodiment of the present disclosure generating the transformed synthetic representation comprises:
- generating a difference representation, wherein generating the difference representation comprises subtracting the native representation from the synthetic representation,
- generating an attenuated difference representation, wherein generating the attenuated difference representation comprises multiplying the difference representation with an attenuation factor,
- adding the attenuated difference representation to the native representation or subtracting the attenuated difference representation from the synthetic representation.

As described, the model is to be made to generate a synthetic representation representing the examination area of the new examination object after application of a second amount of a contrast agent, the second amount being larger than the first amount.

Subtracting the native representation from the synthetic representation results in a difference representation in which non-zero difference values are essentially due to the contrast agent (see, e.g., A. Fringuello Mingo et al.: Amplifying the Effects of Contrast Agents on Magnetic Resonance Images Using a Deep Learning Method Trained on Synthetic Data, Investigative Radiology 58(12), 2023, 853-864). The difference representation is therefore a representation of the contrast agent distribution in the examination area.

By multiplying the difference representation by an attenuation factor, the signal intensity caused by the contrast agent in the examination area is attenuated. The attenuation factor is usually a real number that is greater than zero and less than one.

The result of the multiplication is an attenuated difference representation. The attenuated difference representation can be added to the native representation. The result of this addition is a transformed synthetic representation of the examination area of the new examination object. The result of the addition is a synthetic contrast-enhanced representation of the examination area of the new examination object, in which the contrast enhancement is lower than in the case of the synthetic representation.

The pre-trained conditional generative model is to be made to generate a synthetic representation in which the contrast enhancement after transformation corresponds to the contrast enhancement of the contrast-enhanced representation.

In other words, the model is to be made to generate a synthetic representation which, after transformation, represents the examination area of the new examination object after application of the first amount of contrast agent.

The attenuated difference representation can also be subtracted from the synthetic representation to generate the transformed synthetic representation.

It is also possible to multiply the difference representation or the attenuated difference representation in frequency space by a frequency-dependent weighting function (see, e.g., WO2024052156A1). Such a frequency-dependent weighting function can have the function of a filter. Preferred weight functions are Hann function (also referred to as the Hann window) and Poisson function (Poisson window). Examples of other weight functions can be found for example at https://de.wikipedia.org/wiki/Fensterfunktion#Beispiele_von_Fensterfunktionen; F.J. Harris et al.: On the Use of Windows for Harmonic Analysis with the Discrete Fourier Transform, Proceedings of the IEEE, vol. 66, No. 1, 1978; https://docs.scipy.org/doc/scipy/reference/signal.windows.html; K. M. M Prabhu: Window Functions and Their Applications in Signal Processing, CRC Press, 2014, 978-1-4665-1583-3; WO2024052156A1).

Fig. 3 shows schematically an example of generating a transformed synthetic representation.

The representations R0, SR and SR^{T} shown in Fig. 3 correspond to the representations R0, SR and SR^{T} shown in Fig. 2.

In a first step, the difference representation DR is generated by subtracting the native representation R0 from the synthetic representation SR: DR=SR-R0.

In a further step, the attenuated difference representation aDR is generated by multiplying the difference representation DR with an attenuation factor α: aDR=α·DR. The attenuation factor α is a real number. Usually, the attenuation factor α is greater than zero and less than 1: 0 < α < 1.

In a next step, the transformed synthetic representation SR^{T} is generated. The transformed synthetic representation SR^{T} can be generated by adding the attenuated difference representation aDR to the native representation: SR^{T} =R0+aDR. The transformed synthetic representation SR^{T} can be generated by subtracting the attenuated difference representation aDR from the synthetic representation SR: SR^{T} =SR-aDR.

As described, the generation of the transformed synthetic representation may comprise further steps, such as filtering or the like. It should be noted that Fig. 3 shows only one possibility of a transformation; further possibilities are conceivable.

Fig. 4 shows schematically another example of using a pre-trained conditional generative model to generate a synthetic representation of the examination area of a new examination object.

In a first step, a native representation R0 and a contrast-enhanced representation R1 are received.

The native representation R0 and the contrast-enhanced representation R1 are radiologic representations, such as CT representations or MRI representations.

The native representation R0 represents the examination area of a new examination object without contrast agent. The contrast-enhanced representation R1 represents the same examination area of the same examination object after administration of a first amount of contrast agent. The examination area comprises the lungs of a human.

An encoder E is used to generate one or more embeddings based on the native representation R0 and the first representation R1. As described, it is also possible for one or more embeddings to be generated based on only one of the representations.

In the exampled shown in Fig. 4, the pre-trained conditional generative model CGM^{t} comprises a denoising model DM^{t} of a conditional diffusion model. The pre-trained conditional generative model CGM^{t} may have been pre-trained in a process as described with reference to Fig. 1. In an embodiment of the present disclosure, the conditional generative model CGM^{t} was pre-trained based on representations of human lungs, as the pre-trained conditional generative model CGM^{t} is used in the example shown in Fig. 4 to generate a synthetic representation SR of a human lung.

The pre-trained conditional generative model CGM^{t} is configured and was pre-trained to generate the synthetic representation SR of the examination area of the new examination object based on starting data SD. The starting data SD can be noise data, for example. The synthetic representation SR represents the examination area of the examination object after application of a second amount of a contrast agent, wherein the second amount is larger than the first amount.

The synthetic representation SR is converted into a transformed synthetic representation SR^{T}. The native representation R0 is used for the transformation. The transformed synthetic representation SR^{T} represents the examination area of the examination object after application of the first amount of the contrast agent.

For obtaining the transformed synthetic representation SR^{T} it is possible, for example, to subtract the native representation R0 from the synthetic representation SR, multiply the result of the subtraction by an attenuation factor (e.g., a real number that is greater than zero and less than 1) and either add the result of the multiplication to the native representation R0 or subtract it from the synthetic representation SR to obtain the transformed synthetic representation SR^{T}. Other transformations are also possible.

A loss function LF is used to quantify deviations between the transformed synthetic representation SR^{T} and the contrast-enhanced representation R1. In an optimization process, the starting data SD and/or the one or more embeddings and/or one or more parameters of the pre-trained conditional generative model CGM^{t} are modified to reduce the deviations. The process its repeated one or more times.

Once a stop criterion has been reached, the last synthetic representation generated (or a synthetic image generated therefrom) can be output (e.g., shown on a display and/or printed out on a printer), stored in a data memory and/or transferred to another computer system.

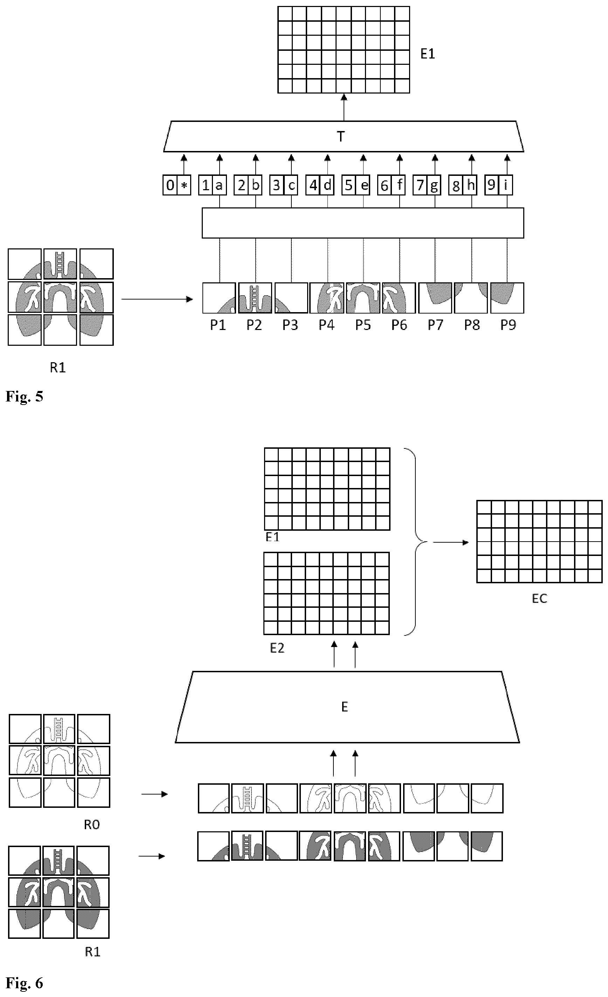

Fig. 5 shows a schematic example of generating an embedding based on a representation of an examination area of an examination object.

The representation R1 is split into fixed-size patches P1 to P9. Patch embeddings *a* to *i* are generated by flattening the patches P1 to P9 and mapping the flattened patches by linear projection to a dimension corresponding to the input dimension of the transformer T. Position embeddings 1 to 9 are added to the patch embeddings to retain positional information. The resulting sequence serves as input to the transformer T.

In the example shown, the sequence is preceded by an embedding * with the position 0, which can contain global information about the representation R1, e.g., which examination region it shows and/or the measurement conditions under which it was generated and/or other/further information.

It is possible that other/further embeddings are added to the sequence. For example, one or more learnable input tokens can be added, that the conditional generative model can use as "registers" (see, e.g., T. Darcet et al.: Vision Transformers Need Registers, arXiv:2309.16588v1).

The transformer T generates an embedding E1 from the sequence.

This process for generating embeddings is known and described, for example, in: A. Dosovitsky et al.: An Image is Worth 16x16 Words: Transformers for Image Recognition at Scale, arXiv:2010.11929v2).

Fig. 6 shows a schematic example of generating several embeddings using an encoder and combining the embeddings into a single embedding.

Fig. 6 shows a native representation R0 and a contrast-enhanced representation R1. The representations R0 and R1 shown in Fig. 6 may correspond to the representations shown in Fig. 4.

The native representation R0 and the contrast-enhanced representation R1 are radiologic representations, such as CT representations or MRI representations.

The native representation R0 represents the examination area of a new examination object without contrast agent. The contrast-enhanced representation R1 represents the same examination area of the same examination object after administration of a first amount of contrast agent. The examination area comprises the lungs of a human.

Patches are generated from each representation R0 and R1, and fed to the encoder E. In the example shown in Fig. 6, the encoder E comprises a transformer.

The encoder E generates a first embedding E1 based on the patches of the native representation R0, and second embedding E2 based on the patches of the contrast-enhanced representation R1. The embeddings can be generated as described in relation to Fig. 5.

The embeddings E1 and E2 are combined into a single image embedding EC. This combined embedding EC can be used as a condition when reconstructing a reference representation and/or when generating a synthetic representation.

The embeddings E1 and E2 can be combined into the single image embedding EC in various ways, e.g., by concatenation, by summing the image embeddings together elementwise, by performing a principal component analysis (PCA) and generating an embedding based on principle components, by taking the element-wise mean (e.g., arithmetic means) of the image embeddings, by taking the element-wise maximum (e.g., arithmetic means) of the image embeddings, by weighted averaging, and/or by using a trainable machine learning model (such as an artificial neural network).

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

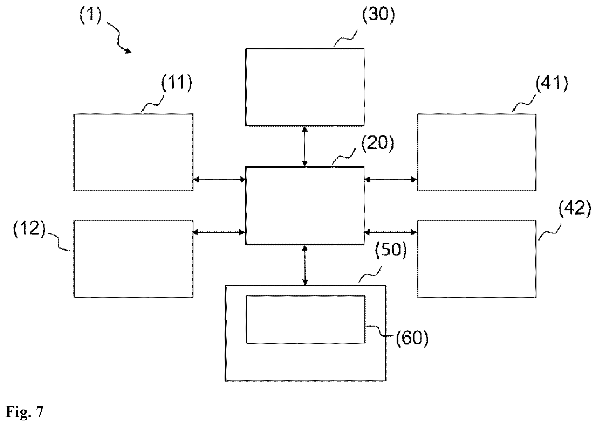

Fig. 7 illustrates a computer system (1) according to some example implementations of the present disclosure in more detail. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), lightemitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

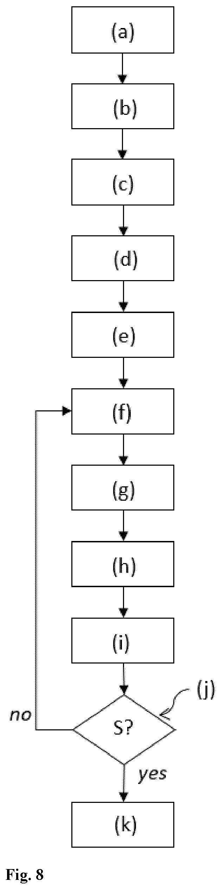

Fig. 8 schematically shows an embodiment of the computer-implemented method for generating a synthetic medical representation in the form of a flow chart. The method comprises the steps:
(a) providing a pre-trained conditional generative model, wherein the pre-trained conditional generative model was trained to reconstruct a plurality of reference representations of an examination area of a plurality of examination objects,
(b) providing a native representation of the examination area of a new examination object, wherein the native representation represents the examination area of the new examination object without contrast agent,
(c) providing a contrast-enhanced representation of the examination area of the new examination object, wherein the contrast-enhanced representation represents the examination area of the new examination object after administration of an amount of a contrast agent,
(d) generating one or more embeddings at least partially based on the native representation and/or the contrast-enhanced representation,
(e) providing starting data,
(f) generating a synthetic representation of the examination area of the new examination object based on the starting data using the pre-trained conditional generative model and using the one or more embeddings as condition,
(g) generating a transformed synthetic representation based on the synthetic representation, wherein the transformed synthetic representation is a contrast-enhanced representation of the examination area of the new examination object in which the contrast-enhancement is reduced compared to the synthetic representation,
(h) quantifying deviations between the transformed synthetic representation and the contrast-enhanced representation of the examination area of the new examination object,
(i) reducing the deviations by modifying the starting data and/or the one or more embeddings and/or parameters of the pre-trained conditional generative model,
(j) repeating steps (f) to (i) until a stop criterion (S) is reached,
(k) outputting and/or storing the synthetic representation of the examination area of the new examination object and/or a synthetic image of the examination area of the new examination object generated therefrom, and/or transmitting the synthetic representation and/or the synthetic image to a separate computer system.

The computer-readable program code of the present disclosure may also be marketed in combination with a contrast agent. Such a combination is also referred to as a kit. Such a kit includes the contrast agent and the computer-readable program code. It is also possible that such a kit includes the contrast agent and means for allowing a purchaser to obtain the computer-readable program code, e.g., download it from an Internet site. These means may include a link, i.e., an address of the Internet site from which the computer-readable program code may be obtained, e.g., from which the computer-readable program code may be downloaded to a computer system connected to the Internet. Such means may include a code (e.g., an alphanumeric string or a QR code, or a DataMatrix code or a barcode or other optically and/or electronically readable code) by which the purchaser can access the computer-readable program code. Such a link and/or code may, for example, be printed on a package of the contrast agent and/or printed on a package insert for the contrast agent. A kit is thus a combination product comprising a contrast agent and a computer-readable program code (e.g., in the form of access to the computer-readable program code or in the form of executable program code on a data carrier) that is offered for sale together.

## Claims

1. A computer-implemented method comprising:
(a) providing a pre-trained conditional generative model (CGM^{t}), wherein the pre-trained conditional generative model (CGM^{t}) was trained to reconstruct a plurality of reference representations (RR1, RR2, RR3, RR4, RR5, RR6) of an examination area of a plurality of examination objects,
(b) providing a native representation (R0) of the examination area of a new examination object, wherein the native representation (R0) represents the examination area of the new examination object without contrast agent,
(c) providing a contrast-enhanced representation (R1) of the examination area of the new examination object, wherein the contrast-enhanced representation (R1) represents the examination area of the new examination object after administration of an amount of a contrast agent,
(d) generating one or more embeddings (E1, E2, EC) at least partially based on the native representation (R0) and/or the contrast-enhanced representation (R1),
(e) providing starting data (SD),
(f) generating a synthetic representation (SR) of the examination area of the new examination object based on the starting data (SD) using the pre-trained conditional generative model (CGM^{t}) and using the one or more embeddings (E1, E2, EC) as condition,
(g) generating a transformed synthetic representation (SR^{T}) based on the synthetic representation (SR), wherein the transformed synthetic representation (SR^{T}) is a contrast-enhanced representation of the examination area of the new examination object in which the contrast-enhancement is reduced compared to the synthetic representation (SR),
(h) quantifying deviations between the transformed synthetic representation (SR^{T}) and the contrast-enhanced representation (R1) of the examination area of the new examination object,
(i) reducing the deviations by modifying the starting data (SD) and/or the one or more embeddings (E1, E2, EC) and/or parameters of the pre-trained conditional generative model (CGM^{t}),
(j) repeating steps (f) to (i) one or more times,
(k) outputting and/or storing the synthetic representation (SR) of the examination area of the new examination object and/or a synthetic image of the examination area of the new examination object generated therefrom, and/or transmitting the synthetic representation (SR) and/or the synthetic image to a separate computer system.

2. The computer-implemented method of claim 1, wherein the pre-trained conditional generative model (CGM^{t}) was pre-trained on training data (TD), wherein the training data (TD) comprised, for each examination object of the plurality of examination objects, one or more reference representations (RR1, RR2, RR3, RR4, RR5, RR6) of the examination area of the examination object, wherein pre-training comprised, for each examination object of the plurality of examination objects:
- generating one or more embeddings (E1, E2, EC) based on the one or more reference representations (RR1, RR2, RR3, RR4, RR5, RR6) of the examination area of the examination object,
- inputting one or more reference representations (RR1, RR2, RR3, RR4, RR5, RR6) of the examination area of the examination object into the conditional generative model (CGM), and reconstruction at least one of the one or more reference representations (RR1, RR2, RR3, RR4, RR5, RR6) by the conditional generative model (CGM) using the one or more embeddings (E1, E2, EC) as condition,
- receiving a reconstructed reference representation (RR1^{R}, RR2^{R}, RR3^{R}, RR4^{R}, RR5^{R}, RR6^{R}) as an output of the conditional generative model (CGM),
- determining deviations between the reconstructed reference representation (RR1^{R}, RR2^{R}, RR3^{R}, RR4^{R}, RR5^{R}, RR6^{R}) and one of the one or more reference representations (RR1, RR2, RR3, RR4, RR5, RR6),
- reducing the deviations by modifying parameters of the conditional generative model (CGM).

3. The computer-implemented method of claim 1 or 2, wherein each examination object is a human, wherein the examination area is or comprises a liver, a kidney, a heart, a lung, a brain, a stomach, a bladder, a prostate, an intestine, an eye, a breast, a thyroid, a pancreas, or an uterus or a part thereof.

4. The computer-implemented method of any one of claims 1 to 3, wherein each representation is a computed tomography representation, an X-ray representation, a magnetic resonance imaging representation, an ultrasound representation, or a positron emission tomography representation.

5. The computer-implemented method of any one of claims 1 to 4, wherein each representation is a representation in real space, in frequency space or in projection space.

6. The computer-implemented method of any one of claims 1 to 5, wherein generating the transformed synthetic representation (SR^{T}) comprises: reducing the contrast-enhancement of the synthetic representation (SR).

7. The computer-implemented method of any one of claims 1 to 6, wherein generating the transformed synthetic representation (SR^{T}) comprises: reducing the contrast-enhancement of the synthetic representation (SR), so that the contrast-enhancement of the transformed synthetic representation (SR^{T}) corresponds to the contrast-enhancement of the contrast-enhanced representation (R1).

8. The computer-implemented method of any one of claims 1 to 7, wherein generating the transformed synthetic representation (SR^{T}) comprises: reducing the contrast-enhancement of the synthetic representation (SR), thereby generating a transformed synthetic representation that represents the examination area of the new examination object after administration of the first amount of contrast agent.

9. The computer-implemented method of any one of claims 1 to 8, wherein generating the transformed synthetic representation (SR^{T}) comprises:
- generating a difference representation (DR), wherein generating the difference representation (DR) comprises subtracting the native representation (R0) from the synthetic representation (SR),
- generating an attenuated difference representation (aDR), wherein generating the attenuated difference representation (aDR) comprises multiplying the difference representation (DR) with an attenuation factor,
- adding the attenuated difference representation (aDR) to the native representation (R0) or subtracting the attenuated difference representation (aDR) from the synthetic representation (SR).

10. The computer-implemented method of claim 9, wherein generating the transformed synthetic representation (SR^{T}) further comprises: multiplying the difference representation (DR) or the attenuated difference representation (aDR) in frequency space by a frequency-dependent weighting function.

11. The computer-implemented method of any one of claims 1 to 10, wherein steps (f) to (i) are repeated until a stop criterion is reached, wherein the stop criterion comprises: a predefined maximum number of steps has been performed, the deviations between the transformed synthetic representation (SR^{T}) and the contrast-enhanced representation (R1) can no longer be reduced by modifying the starting data (SD) and/or the one or more embeddings (E1, E2, EC) and/or the model parameters, a predefined minimum of a loss function (LF) is reached, and/or an extreme value of another performance value is reached.

12. The computer-implemented method of any one of claims 1 to 11, wherein the pre-trained conditional generative model (CGM^{t}) is or comprises a diffusion model (DM^{t}) or a portion thereof.

13. The computer-implemented method of any one of claims 1 to 12, wherein the one or more embeddings (E1, E2, EC) are generated using one or more encoders (E), wherein the one or more encoders (E) comprise one or more transformers and/or convolutional neural networks.

14. A computer system comprising:
a processing unit (20); and
a memory (50) storing a computer program (60) configured to perform, when executed by the processing unit (20), an operation, the operation comprising:
(a) providing a pre-trained conditional generative model (CGM^{t}), wherein the pre-trained conditional generative model (CGM^{t}) was trained to reconstruct a plurality of reference representations (RR1, RR2, RR3, RR4, RR5, RR6) of an examination area of a plurality of examination objects,
(b) providing a native representation (R0) of the examination area of a new examination object, wherein the native representation (R0) represents the examination area of the new examination object without contrast agent,
(c) providing a contrast-enhanced representation (R1) of the examination area of the new examination object, wherein the contrast-enhanced representation (R1) represents the examination area of the new examination object after administration of an amount of a contrast agent,
(d) generating one or more embeddings (E1, E2, EC) at least partially based on the native representation (R0) and/or the contrast-enhanced representation (R1),
(e) providing starting data (SD),
(f) generating a synthetic representation (SR) of the examination area of the new examination object based on the starting data (SD) using the pre-trained conditional generative model (CGM^{t}) and using the one or more embeddings (E1, E2, EC) as condition,
(g) generating a transformed synthetic representation (SR^{T}) based on the synthetic representation (SR), wherein the transformed synthetic representation (SR^{T}) is a contrast-enhanced representation of the examination area of the new examination object in which the contrast-enhancement is reduced compared to the synthetic representation (SR),
(h) quantifying deviations between the transformed synthetic representation (SR^{T}) and the contrast-enhanced representation (R1) of the examination area of the new examination object,
(i) reducing the deviations by modifying the starting data (SD) and/or the one or more embeddings (E1, E2, EC) and/or parameters of the pre-trained conditional generative model (CGM^{t}),
(j) repeating steps (f) to (i) one or more times,
(k) outputting and/or storing the synthetic representation (SR) of the examination area of the new examination object and/or a synthetic image of the examination area of the new examination object generated therefrom, and/or transmitting the synthetic representation (SR) and/or the synthetic image to a separate computer system.

15. A non-transitory computer readable storage medium having stored thereon software instructions that, when executed by a processing unit (20) of a computer system (1), cause the computer system (1) to perform the following steps:
(a) providing a pre-trained conditional generative model (CGM^{t}), wherein the pre-trained conditional generative model (CGM^{t}) was trained to reconstruct a plurality of reference representations (RR1, RR2, RR3, RR4, RR5, RR6) of an examination area of a plurality of examination objects,
(b) providing a native representation (R0) of the examination area of a new examination object, wherein the native representation (R0) represents the examination area of the new examination object without contrast agent,
(c) providing a contrast-enhanced representation (R1) of the examination area of the new examination object, wherein the contrast-enhanced representation (R1) represents the examination area of the new examination object after administration of an amount of a contrast agent,
(d) generating one or more embeddings (E1, E2, EC) at least partially based on the native representation (R0) and/or the contrast-enhanced representation (R1),
(e) providing starting data (SD),
(f) generating a synthetic representation (SR) of the examination area of the new examination object based on the starting data (SD) using the pre-trained conditional generative model (CGM^{t}) and using the one or more embeddings (E1, E2, EC) as condition,
(g) generating a transformed synthetic representation (SR^{T}) based on the synthetic representation (SR), wherein the transformed synthetic representation (SR^{T}) is a contrast-enhanced representation of the examination area of the new examination object in which the contrast-enhancement is reduced compared to the synthetic representation (SR),
(h) quantifying deviations between the transformed synthetic representation (SR^{T}) and the contrast-enhanced representation (R1) of the examination area of the new examination object,
(i) reducing the deviations by modifying the starting data (SD) and/or the one or more embeddings (E1, E2, EC) and/or parameters of the pre-trained conditional generative model (CGM^{t}),
(j) repeating steps (f) to (i) one or more times,
(k) outputting and/or storing the synthetic representation (SR) of the examination area of the new examination object and/or a synthetic image of the examination area of the new examination object generated therefrom, and/or transmitting the synthetic representation (SR) and/or the synthetic image to a separate computer system.

16. Use of a contrast agent in an examination of an examination area of a new examination object, the examination comprising:
(a) providing a pre-trained conditional generative model (CGM^{t}), wherein the pre-trained conditional generative model (CGM^{t}) was trained to reconstruct a plurality of reference representations (RR1, RR2, RR3, RR4, RR5, RR6) of an examination area of a plurality of examination objects,
(b) generating a native representation (R0) of the examination area of the new examination object, wherein the native representation (R0) represents the examination area of the new examination object without contrast agent,
(c) generating a contrast-enhanced representation (R1) of the examination area of the new examination object, wherein the contrast-enhanced representation (R1) represents the examination area of the new examination object after administration of an amount of the contrast agent,
(d) generating one or more embeddings (E1, E2, EC) at least partially based on the native representation (R0) and/or the contrast-enhanced representation (R1),
(e) providing starting data (SD),
(f) generating a synthetic representation (SR) of the examination area of the new examination object based on the starting data (SD) using the pre-trained conditional generative model (CGM^{t}) and using the one or more embeddings (E1, E2, EC) as condition,
(g) generating a transformed synthetic representation (SR^{T}) based on the synthetic representation (SR), wherein the transformed synthetic representation (SR^{T}) is a contrast-enhanced representation of the examination area of the new examination object in which the contrast-enhancement is reduced compared to the synthetic representation (SR),
(h) quantifying deviations between the transformed synthetic representation (SR^{T}) and the contrast-enhanced representation (R1) of the examination area of the new examination object,
(i) reducing the deviations by modifying the starting data (SD) and/or the one or more embeddings (E1, E2, EC) and/or parameters of the pre-trained conditional generative model (CGM^{t}),
(j) repeating steps (f) to (i) one or more times,
(k) outputting and/or storing the synthetic representation (SR) of the examination area of the new examination object and/or a synthetic image of the examination area of the new examination object generated therefrom, and/or transmitting the synthetic representation (SR) and/or the synthetic image to a separate computer system.

17. Use of claim 16, wherein the contrast agent is or comprises:
gadolinium 2,2',2"-(10-{1-carboxy-2-[2-(4-ethoxyphenyl)ethoxy]ethyl} -1,4,7,10-tetraazacyclododecane-1,4,7-triyl)triacetate,
gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate,
gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
gadolinium (2S,2'S,2"S)-2,2',2"-{10-[(1S)-1-carboxy-4-14-[2-(2-ethoxyethoxy)ethoxy]phenyl}butyl]-1,4,7,10-tetraazacyclododecane-1,4,7-triyl}tris(3-hydroxypropanoate,
gadolinium 2,2',2"-{10-[(1S)-4-(4-butoxyphenyl)-1-carboxybutyl]-1,4,7,10-tetraazacyclododecane-1,4,7-triyl}triacetate,
gadolinium -2,2',2"-{(2S)-10-(carboxymethyl)-2-[4-(2-ethoxyethoxy)benzyl]-1,4,7,10-tetraazacyclododecane-1,4,7-triyl}triacetate,
gadolinium 2,2',2"-[10-(carboxymethyl)-2-(4-ethoxybenzyl)-1,4,7,10-tetraazacyclododecane-1,4,7-triyl]triacetate.
gadolinium(III) 5,8-bis(carboxylatomethyl)-2-[2-(methylamino)-2-oxoethyl]-10-oxo-2,5,8,11-tetraazadodecane-1-carboxylate hydrate,
gadolinium(III) 2-[4-(2-hydroxypropyl)-7,10-bis(2-oxido-2-oxoethyl)-1,4,7,10-tetrazacyclododec-1-yl]acetate,
gadolinium(III) 2,2',2"-(10-((2R,3S)-1,3,4-trihydroxybutan-2-yl)-1,4,7,10-tetraazacyclododecane-1,4,7-triyl)triacetate,
gadolinium(III) 2-[4,7,10-tris(carboxymethyl)-1,4,7,10-tetrazacyclododec-1-yl]acetic acid,
gadolinium(III) ethoxybenzyldiethylenetriaminepentaacetic acid,
gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate,
dihydrogen [(±)-4-carboxy-5,8,11-tris(carboxymethyl)-1-phenyl-2-oxa-5,8,11-triazatridecan-13-oato(5-)]gadolinate(2-),
tetragadolinium [4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetate,
a Gd³⁺ complex of a compound of the formula (I) 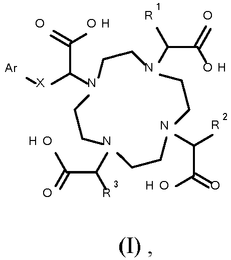
where
Ar is a group selected from 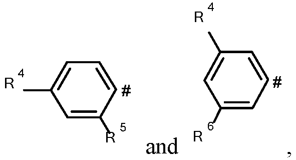
where # is the linkage to X,
X is a group selected from
CH₂, (CH₂)₂, (CH₂)₃, (CH₂)₄ and *-(CH₂)₂-O-CH₂-^{#} ,
where * is the linkage to Ar and ^{#} is the linkage to the acetic acid residue,
R¹, R² and R³ are each independently a hydrogen atom or a group selected from C₁-C₃ alkyl, -CH₂OH, -(CH₂)₂OH and -CH₂OCH₃,
R⁴ is a group selected from C₂-C₄ alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ is a hydrogen atom,
and
R⁶ is a hydrogen atom,
or a stereoisomer, tautomer, hydrate, solvate or salt thereof, or a mixture thereof, or
a Gd³⁺ complex of a compound of the formula (II) 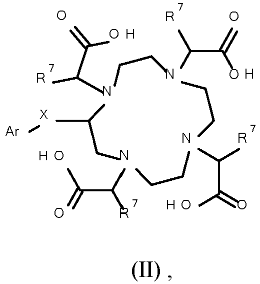
where
Ar is a group selected from 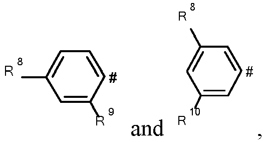
where # is the linkage to X,
X is a group selected from CH₂, (CH₂)₂, (CH₂)₃, (CH₂)₄ and *-(CH₂)₂-O-CH₂-^{#}, where * is the linkage to Ar and ^{#} is the linkage to the acetic acid residue,
R⁷ is a hydrogen atom or a group selected from C₁-C₃ alkyl, -CH₂OH,
-(CH₂)₂OH and -CH₂OCH₃;
R⁸ is a group selected from
C₂-C₄ alkoxy, (H₃C-CH₂O)-(CH₂)₂-O-, (H₃C-CH₂O)-(CH₂)₂-O-(CH₂)₂-O- and
(H₃C-CH₂O)-(CH₂)₂-O-(CH₂)₂-O-(CH₂)₂-O-;
R⁹ and R¹⁰ independently represent a hydrogen atom;
or a stereoisomer, tautomer, hydrate, solvate or salt thereof, or a mixture thereof.

18. A kit comprising a contrast agent and computer-readable program code that, when executed by a processing unit (20) of a computer system (1), cause the computer system (1) to execute the following steps:
(a) providing a pre-trained conditional generative model (CGM^{t}), wherein the pre-trained conditional generative model (CGM^{t}) was trained to reconstruct a plurality of reference representations (RR1, RR2, RR3, RR4, RR5, RR6) of an examination area of a plurality of examination objects,
(b) providing a native representation of the examination area of a new examination object, wherein the native representation represents the examination area of the new examination object without contrast agent,
(c) providing a contrast-enhanced representation (R1) of the examination area of the new examination object, wherein the contrast-enhanced representation (R1) represents the examination area of the new examination object after administration of an amount of the contrast agent,
(d) generating one or more embeddings (E1, E2, EC) at least partially based on the native representation and/or the contrast-enhanced representation (R1),
(e) providing starting data (SD),
(f) generating a synthetic representation (SR) of the examination area of the new examination object based on the starting data (SD) using the pre-trained conditional generative model (CGM^{t}) and using the one or more embeddings (E1, E2, EC) as condition,
(g) generating a transformed synthetic representation (SR^{T}) based on the synthetic representation (SR), wherein the transformed synthetic representation (SR^{T}) is a contrast-enhanced representation of the examination area of the new examination object in which the contrast-enhancement is reduced compared to the synthetic representation (SR),
(h) quantifying deviations between the transformed synthetic representation (SR^{T}) and the contrast-enhanced representation (R1) of the examination area of the new examination object,
(i) reducing the deviations by modifying the starting data (SD) and/or the one or more embeddings (E1, E2, EC) and/or parameters of the pre-trained conditional generative model (CGM^{t}),
(j) repeating steps (f) to (i) one or more times,
(k) outputting and/or storing the synthetic representation (SR) of the examination area of the new examination object and/or a synthetic image of the examination area of the new examination object generated therefrom, and/or transmitting the synthetic representation (SR) and/or the synthetic image to a separate computer system.

## Patentansprüche

1. Computerimplementiertes Verfahren, umfassend:
(a) Bereitstellen eines vortrainierten bedingten generativen Modells (CGM^{t}), wobei das vortrainierte bedingte generative Modell (CGM^{t}) trainiert wurde, um eine Mehrzahl von Referenzdarstellungen (RR1, RR2, RR3, RR4, RR5, RR6) eines Untersuchungsbereichs einer Mehrzahl von Untersuchungsobjekten zu rekonstruieren,
(b) Bereitstellen einer nativen Darstellung (R0) des Untersuchungsbereichs eines neuen Untersuchungsobjekts, wobei die native Darstellung (R0) den Untersuchungsbereich des neuen Untersuchungsobjekts ohne Kontrastmittel darstellt,
(c) Bereitstellen einer kontrastverstärkten Darstellung (R1) des Untersuchungsbereichs des neuen Untersuchungsobjekts, wobei die kontrastverstärkte Darstellung (R1) den Untersuchungsbereich des neuen Untersuchungsobjekts nach Verabreichung einer Menge eines Kontrastmittels darstellt,
(d) Erzeugen einer oder mehrerer Einbettungen (E1, E2, EC) mindestens teilweise basierend auf der nativen Darstellung (R0) und/oder der kontrastverstärkten Darstellung (R1),
(e) Bereitstellen von Anfangsdaten (SD),
(f) Erzeugen einer synthetischen Darstellung (SR) des Untersuchungsbereichs des neuen Untersuchungsobjekts basierend auf den Anfangsdaten (SD) unter Verwendung des vortrainierten bedingten generativen Modells (CGM^{t}) und unter Verwendung der einen oder der mehreren Einbettungen (E1, E2, EC) als Bedingung,
(g) Erzeugen einer transformierten synthetischen Darstellung (SR^{T}) basierend auf der synthetischen Darstellung (SR), wobei die transformierte synthetische Darstellung (SR^{T}) eine kontrastverstärkte Darstellung des Untersuchungsbereichs des neuen Untersuchungsobjekts ist, in dem die Kontrastverstärkung im Vergleich zu der synthetischen Darstellung (SR) reduziert ist,
(h) Quantifizieren von Abweichungen zwischen der transformierten synthetischen Darstellung (SR^{T}) und der kontrastverstärkten Darstellung (R1) des Untersuchungsbereichs des neuen Untersuchungsobjekts,
(i) Reduzieren der Abweichungen durch Modifizieren der Anfangsdaten (SD) und/oder der einen oder der mehreren Einbettungen (E1, E2, EC) und/oder von Parametern des vortrainierten bedingten generativen Modells (CGM^{t}),
(j) ein- oder mehrmaliges Wiederholen der Schritte (f) bis (i),
(k) Ausgeben und/oder Speichern der synthetischen Darstellung (SR) des Untersuchungsbereichs des neuen Untersuchungsobjekts und/oder eines daraus erzeugten synthetischen Bildes des Untersuchungsbereichs des neuen Untersuchungsobjekts und/oder Übertragen der synthetischen Darstellung (SR) und/oder des synthetischen Bildes an ein separates Computersystem.

2. Computerimplementiertes Verfahren nach Anspruch 1, wobei das vortrainierte bedingte generative Modell (CGM^{t}) mit Trainingsdaten (TD) vortrainiert wurde, wobei die Trainingsdaten (TD) für jedes Untersuchungsobjekt der Mehrzahl von Untersuchungsobjekten eine oder mehrere Referenzdarstellungen (RR1, RR2, RR3, RR4, RR5, RR6) des Untersuchungsbereichs des Untersuchungsobjekts umfassten, wobei das Vortrainieren für jedes Untersuchungsobjekt der Mehrzahl von Untersuchungsobjekten Folgendes umfasste:
- Erzeugen einer oder mehrerer Einbettungen (E1, E2, EC) basierend auf der einen oder den mehreren Referenzdarstellungen (RR1, RR2, RR3, RR4, RR5, RR6) des Untersuchungsbereichs des Untersuchungsobjekts,
- Eingeben einer oder mehrerer Referenzdarstellungen (RR1, RR2, RR3, RR4, RR5, RR6) des Untersuchungsbereichs des Untersuchungsobjekts in das bedingte generative Modell (CGM) und Rekonstruieren mindestens einer der einen oder der mehreren Referenzdarstellungen (RR1, RR2, RR3, RR4, RR5, RR6) durch das bedingte generative Modell (CGM) unter Verwendung der einen oder mehreren Einbettungen (E1, E2, EC) als Bedingung,
- Empfangen einer rekonstruierten Referenzdarstellung (RR1^{R}, RR2^{R}, RR3^{R}, RR4^{R}, RR5^{R}, RR6^{R}) als Ausgabe des bedingten generativen Modells (CGM),
- Bestimmen von Abweichungen zwischen der rekonstruierten Referenzdarstellung (RR1^{R}, RR2^{R}, RR3^{R}, RR4^{R}, RR5^{R}, RR6^{R}) und einer der einen oder der mehreren Referenzdarstellungen (RR1, RR2, RR3, RR4, RR5, RR6),
- Reduzieren der Abweichungen durch Modifizieren von Parametern des bedingten generativen Modells (CGM).

3. Computerimplementiertes Verfahren nach Anspruch 1 oder 2, wobei es sich bei jedem Untersuchungsobjekt um einen Menschen handelt, wobei der Untersuchungsbereich eine Leber, eine Niere, ein Herz, eine Lunge, ein Gehirn, ein(en) Magen, eine Blase, eine Prostata, ein(en) Darm, ein Auge, eine Brust, eine Schilddrüse, eine Bauchspeicheldrüse oder eine Gebärmutter oder ein(en) Teil davon ist oder umfasst.

4. Computerimplementiertes Verfahren nach einem der Ansprüche 1 bis 3, wobei jede Darstellung eine Computertomographie-Darstellung, eine Röntgendarstellung, eine Magnetresonanzbildgebungsdarstellung, eine Ultraschalldarstellung oder eine Positronenemissionstomographie-Darstellung ist.

5. Computerimplementiertes Verfahren nach einem der Ansprüche 1 bis 4, wobei jede Darstellung eine Darstellung im realen Raum, im Frequenzraum oder im Projektionsraum ist.

6. Computerimplementiertes Verfahren nach einem der Ansprüche 1 bis 5, wobei das Erzeugen der transformierten synthetischen Darstellung (SR^{T}) Folgendes umfasst: Reduzieren der Kontrastverstärkung der synthetischen Darstellung (SR).

7. Computerimplementiertes Verfahren nach einem der Ansprüche 1 bis 6, wobei das Erzeugen der transformierten synthetischen Darstellung (SR^{T}) Folgendes umfasst: Reduzieren der Kontrastverstärkung der synthetischen Darstellung (SR), so dass die Kontrastverstärkung der transformierten synthetischen Darstellung (SR^{T}) der Kontrastverstärkung der kontrastverstärkten Darstellung (R1) entspricht.

8. Computerimplementiertes Verfahren nach einem der Ansprüche 1 bis 7, wobei das Erzeugen der transformierten synthetischen Darstellung (SR^{T}) Folgendes umfasst: Reduzieren der Kontrastverstärkung der synthetischen Darstellung (SR), wodurch eine transformierte synthetische Darstellung erzeugt wird, die den Untersuchungsbereich des neuen Untersuchungsobjekts nach Verabreichung der ersten Menge an Kontrastmittel darstellt.

9. Computerimplementiertes Verfahren nach einem der Ansprüche 1 bis 8, wobei das Erzeugen der transformierten synthetischen Darstellung (SR^{T}) Folgendes umfasst:
- Erzeugen einer Differenzdarstellung (DR), wobei das Erzeugen der Differenzdarstellung (DR) Subtrahieren der nativen Darstellung (R0) von der synthetischen Darstellung (SR) umfasst,
- Erzeugen einer gedämpften Differenzdarstellung (aDR), wobei das Erzeugen der gedämpften Differenzdarstellung (aDR) Multiplizieren der Differenzdarstellung (DR) mit einem Dämpfungsfaktor umfasst,
- Addieren der gedämpften Differenzdarstellung (aDR) zu der nativen Darstellung (R0) oder Subtrahieren der gedämpften Differenzdarstellung (aDR) von der synthetischen Darstellung (SR).

10. Computerimplementiertes Verfahren nach Anspruch 9, wobei das Erzeugen der transformierten synthetischen Darstellung (SR^{T}) ferner Folgendes umfasst: Multiplizieren der Differenzdarstellung (DR) oder der gedämpften Differenzdarstellung (aDR) im Frequenzraum mit einer frequenzabhängigen Gewichtungsfunktion.

11. Computerimplementiertes Verfahren nach einem der Ansprüche 1 bis 10, wobei die Schritte (f) bis (i) wiederholt werden, bis ein Abbruchkriterium erreicht wird, wobei das Abbruchkriterium Folgendes umfasst: es wurde eine vordefinierte maximale Anzahl von Schritten ausgeführt, die Abweichungen zwischen der transformierten synthetischen Darstellung (SR^{T}) und der kontrastverstärkten Darstellung (R1) können durch Modifizieren der Anfangsdaten (SD) und/oder der einen oder mehreren Einbettungen (E1, E2, EC) und/oder der Modellparameter nicht mehr reduziert werden, ein vordefiniertes Minimum einer Verlustfunktion (LF) wird erreicht und/oder ein Extremwert eines anderen Leistungswerts wird erreicht.

12. Computerimplementiertes Verfahren nach einem der Ansprüche 1 bis 11, wobei das vortrainierte bedingte generative Modell (CGM^{t}) ein Diffusionsmodell (DM^{t}) oder ein(en) Teil davon ist oder umfasst.

13. Computerimplementiertes Verfahren nach einem der Ansprüche 1 bis 12, wobei die eine oder die mehreren Einbettungen (E1, E2, EC) unter Verwendung eines oder mehrerer Encoder (E) erzeugt werden, wobei der eine oder die mehreren Encoder (E) einen oder mehrere Transformatoren und/oder neuronale Faltungsnetze umfassen.

14. Computersystem, umfassend:
eine Verarbeitungseinheit (20); und
einen Speicher (50), der ein Computerprogramm (60) speichert, das dazu ausgelegt ist, bei Ausführung durch die Verarbeitungseinheit (20) eine Operation auszuführen, wobei die Operation Folgendes umfasst:
(a) Bereitstellen eines vortrainierten bedingten generativen Modells (CGM^{t}), wobei das vortrainierte bedingte generative Modell (CGM^{t}) trainiert wurde, um eine Mehrzahl von Referenzdarstellungen (RR1, RR2, RR3, RR4, RR5, RR6) eines Untersuchungsbereichs einer Mehrzahl von Untersuchungsobjekten zu rekonstruieren,
(b) Bereitstellen einer nativen Darstellung (R0) des Untersuchungsbereichs eines neuen Untersuchungsobjekts, wobei die native Darstellung (R0) den Untersuchungsbereich des neuen Untersuchungsobjekts ohne Kontrastmittel darstellt,
(c) Bereitstellen einer kontrastverstärkten Darstellung (R1) des Untersuchungsbereichs des neuen Untersuchungsobjekts, wobei die kontrastverstärkte Darstellung (R1) den Untersuchungsbereich des neuen Untersuchungsobjekts nach Verabreichung einer Menge eines Kontrastmittels darstellt,
(d) Erzeugen einer oder mehrerer Einbettungen (E1, E2, EC) mindestens teilweise basierend auf der nativen Darstellung (R0) und/oder der kontrastverstärkten Darstellung (R1),
(e) Bereitstellen von Anfangsdaten (SD),
(f) Erzeugen einer synthetischen Darstellung (SR) des Untersuchungsbereichs des neuen Untersuchungsobjekts basierend auf den Anfangsdaten (SD) unter Verwendung des vortrainierten bedingten generativen Modells (CGM^{t}) und unter Verwendung der einen oder der mehreren Einbettungen (E1, E2, EC) als Bedingung,
(g) Erzeugen einer transformierten synthetischen Darstellung (SR^{T}) basierend auf der synthetischen Darstellung (SR), wobei die transformierte synthetische Darstellung (SR^{T}) eine kontrastverstärkte Darstellung des Untersuchungsbereichs des neuen Untersuchungsobjekts ist, in dem die Kontrastverstärkung im Vergleich zu der synthetischen Darstellung (SR) reduziert ist,
(h) Quantifizieren von Abweichungen zwischen der transformierten synthetischen Darstellung (SR^{T}) und der kontrastverstärkten Darstellung (R1) des Untersuchungsbereichs des neuen Untersuchungsobjekts,
(i) Reduzieren der Abweichungen durch Modifizieren der Anfangsdaten (SD) und/oder der einen oder der mehreren Einbettungen (E1, E2, EC) und/oder von Parametern des vortrainierten bedingten generativen Modells (CGM^{t}),
(j) ein- oder mehrmaliges Wiederholen der Schritte (f) bis (i),
(k) Ausgeben und/oder Speichern der synthetischen Darstellung (SR) des Untersuchungsbereichs des neuen Untersuchungsobjekts und/oder eines daraus erzeugten synthetischen Bildes des Untersuchungsbereichs des neuen Untersuchungsobjekts und/oder Übertragen der synthetischen Darstellung (SR) und/oder des synthetischen Bildes an ein separates Computersystem.

15. Nichtflüchtiges, computerlesbares Speichermedium mit darauf gespeicherten Softwareanweisungen, die bei Ausführung durch eine Verarbeitungseinheit (20) eines Computersystems (1) das Computersystem (1) veranlassen, die folgenden Schritte auszuführen:
(a) Bereitstellen eines vortrainierten bedingten generativen Modells (CGM^{t}), wobei das vortrainierte bedingte generative Modell (CGM^{t}) trainiert wurde, um eine Mehrzahl von Referenzdarstellungen (RR1, RR2, RR3, RR4, RR5, RR6) eines Untersuchungsbereichs einer Mehrzahl von Untersuchungsobjekten zu rekonstruieren,
(b) Bereitstellen einer nativen Darstellung (R0) des Untersuchungsbereichs eines neuen Untersuchungsobjekts, wobei die native Darstellung (R0) den Untersuchungsbereich des neuen Untersuchungsobjekts ohne Kontrastmittel darstellt,
(c) Bereitstellen einer kontrastverstärkten Darstellung (R1) des Untersuchungsbereichs des neuen Untersuchungsobjekts, wobei die kontrastverstärkte Darstellung (R1) den Untersuchungsbereich des neuen Untersuchungsobjekts nach Verabreichung einer Menge eines Kontrastmittels darstellt,
(d) Erzeugen einer oder mehrerer Einbettungen (E1, E2, EC) mindestens teilweise basierend auf der nativen Darstellung (R0) und/oder der kontrastverstärkten Darstellung (R1),
(e) Bereitstellen von Anfangsdaten (SD),
(f) Erzeugen einer synthetischen Darstellung (SR) des Untersuchungsbereichs des neuen Untersuchungsobjekts basierend auf den Anfangsdaten (SD) unter Verwendung des vortrainierten bedingten generativen Modells (CGM^{t}) und unter Verwendung der einen oder der mehreren Einbettungen (E1, E2, EC) als Bedingung,
(g) Erzeugen einer transformierten synthetischen Darstellung (SR^{T}) basierend auf der synthetischen Darstellung (SR), wobei die transformierte synthetische Darstellung (SR^{T}) eine kontrastverstärkte Darstellung des Untersuchungsbereichs des neuen Untersuchungsobjekts ist, in dem die Kontrastverstärkung im Vergleich zu der synthetischen Darstellung (SR) reduziert ist,
(h) Quantifizieren von Abweichungen zwischen der transformierten synthetischen Darstellung (SR^{T}) und der kontrastverstärkten Darstellung (R1) des Untersuchungsbereichs des neuen Untersuchungsobjekts,
(i) Reduzieren der Abweichungen durch Modifizieren der Anfangsdaten (SD) und/oder der einen oder der mehreren Einbettungen (E1, E2, EC) und/oder von Parametern des vortrainierten bedingten generativen Modells (CGM^{t}),
(j) ein- oder mehrmaliges Wiederholen der Schritte (f) bis (i),
(k) Ausgeben und/oder Speichern der synthetischen Darstellung (SR) des Untersuchungsbereichs des neuen Untersuchungsobjekts und/oder eines daraus erzeugten synthetischen Bildes des Untersuchungsbereichs des neuen Untersuchungsobjekts und/oder Übertragen der synthetischen Darstellung (SR) und/oder des synthetischen Bildes an ein separates Computersystem.

16. Verwendung eines Kontrastmittels bei einer Untersuchung eines Untersuchungsbereichs eines neuen Untersuchungsobjekts, wobei die Untersuchung Folgendes umfasst:
(a) Bereitstellen eines vortrainierten bedingten generativen Modells (CGM^{t}), wobei das vortrainierte bedingte generative Modell (CGM^{t}) trainiert wurde, um eine Mehrzahl von Referenzdarstellungen (RR1, RR2, RR3, RR4, RR5, RR6) eines Untersuchungsbereichs einer Mehrzahl von Untersuchungsobjekten zu rekonstruieren,
(b) Erzeugen einer nativen Darstellung (R0) des Untersuchungsbereichs des neuen Untersuchungsobjekts, wobei die native Darstellung (R0) den Untersuchungsbereich des neuen Untersuchungsobjekts ohne Kontrastmittel darstellt,
(c) Erzeugen einer kontrastverstärkten Darstellung (R1) des Untersuchungsbereichs des neuen Untersuchungsobjekts, wobei die kontrastverstärkte Darstellung (R1) den Untersuchungsbereich des neuen Untersuchungsobjekts nach Verabreichung einer Menge des Kontrastmittels darstellt,
(d) Erzeugen einer oder mehrerer Einbettungen (E1, E2, EC) mindestens teilweise basierend auf der nativen Darstellung (R0) und/oder der kontrastverstärkten Darstellung (R1),
(e) Bereitstellen von Anfangsdaten (SD),
(f) Erzeugen einer synthetischen Darstellung (SR) des Untersuchungsbereichs des neuen Untersuchungsobjekts basierend auf den Anfangsdaten (SD) unter Verwendung des vortrainierten bedingten generativen Modells (CGM^{t}) und unter Verwendung der einen oder der mehreren Einbettungen (E1, E2, EC) als Bedingung,
(g) Erzeugen einer transformierten synthetischen Darstellung (SR^{T}) basierend auf der synthetischen Darstellung (SR), wobei die transformierte synthetische Darstellung (SR^{T}) eine kontrastverstärkte Darstellung des Untersuchungsbereichs des neuen Untersuchungsobjekts ist, in dem die Kontrastverstärkung im Vergleich zu der synthetischen Darstellung (SR) reduziert ist,
(h) Quantifizieren von Abweichungen zwischen der transformierten synthetischen Darstellung (SR^{T}) und der kontrastverstärkten Darstellung (R1) des Untersuchungsbereichs des neuen Untersuchungsobjekts,
(i) Reduzieren der Abweichungen durch Modifizieren der Anfangsdaten (SD) und/oder der einen oder der mehreren Einbettungen (E1, E2, EC) und/oder von Parametern des vortrainierten bedingten generativen Modells (CGM^{t}),
(j) ein- oder mehrmaliges Wiederholen der Schritte (f) bis (i),
(k) Ausgeben und/oder Speichern der synthetischen Darstellung (SR) des Untersuchungsbereichs des neuen Untersuchungsobjekts und/oder eines daraus erzeugten synthetischen Bildes des Untersuchungsbereichs des neuen Untersuchungsobjekts und/oder Übertragen der synthetischen Darstellung (SR) und/oder des synthetischen Bildes an ein separates Computersystem.

17. Verwendung nach Anspruch 16, wobei das Kontrastmittel Folgendes ist oder umfasst:
Gadolinium-2,2',2"-(10-{1-carboxy-2-[2-(4-ethoxyphenyl)ethoxy]ethyl}-1,4,7,10-tetraazacyclododecan-1,4,7-triyl)triacetat,
Gadolinium-2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
Gadolinium-2,2',2"-{10-[(1S)-1-carboxy-2-{4-[2-(2-ethoxyethoxy)ethoxy]phenyl}ethyl]-1,4,7,10-tetraazacyclododecan-1,4,7-triyl}triacetat,
Gadolinium-(2S,2'S,2"S)-2,2',2"-{10-[(1S)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-2,2',2"-{10-[(1S)-4-(4-butoxyphenyl)-1-carboxybutyl]-1,4,7,10-tetraazacyclododecan-1,4,7-triyl}triacetat,
Gadolinium-2,2',2"-{ (2S)-10-(carboxymethyl)-2-[4-(2-ethoxyethoxy)benzyl]-1,4,7,10-tetraazacyclododecan-1,4,7-triyl}triacetat,
Gadolinium-2,2',2"-[10-(carboxymethyl)-2-(4-ethoxybenzyl)-1,4,7,10-tetraazacyclododecan-1,4,7-triyl]triacetat,
Gadolinium(III)-5,8-bis(carboxylatomethyl)-2-[2-(methylamino)-2-oxoethyl]-10-oxo-2,5,8,11-tetraazadodecan-1-carboxylathydrat,
Gadolinium(III)-2-[4-(2-hydroxypropyl)-7,10-bis(2-oxido-2-oxoethyl)-1,4,7,10-tetrazacyclododec-1-yl]acetat,
Gadolinium(III)-2,2',2"-(10-((2R,3S)-1,3,4-trihydroxybutan-2-yl)-1,4,7,10-tetraazacyclododecan-1,4,7-triyl)triacetat,
Gadolinium(III)-2-[4,7,10-tris(carboxymethyl)-1,4,7,10-tetrazacyclododec-1-yl]essigsäure,
Gadolinium(III)-ethoxybenzyldiethylentriaminpentaessigsäure,
Gadolinium(III)-2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
Dihydrogen-[(±)-4-carboxy-5,8,11-tris(carboxymethyl)-1-phenyl-2-oxa-5,8,11-triazatridecan-13-oato(5-)]gadolinat(2-),
Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat,
einen Gd³⁺-Komplex einer Verbindung der folgenden Formel (I) : 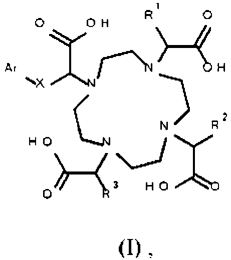
wobei
Ar für eine Gruppe steht, die ausgewählt ist aus 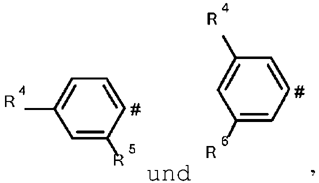
wobei # die Bindung an X ist,
X für eine Gruppe steht, die ausgewählt ist aus CH₂, (CH₂)₂, (CH₂)₃, (CH₂)₄ und *- (CH₂)₂-O-CH₂-^{#},
wobei * die Bindung an Ar ist und ^{#} die Bindung an den Essigsäurerest ist,
R¹, R² and R³ jeweils unabhängig für ein Wasserstoffatom oder eine Gruppe stehen, die ausgewählt ist aus C₁-C₃-Alkyl, -CH₂OH, - (CH₂)₂OH und -CH₂OCH₃,
R⁴ für eine Gruppe steht, die ausgewählt ist aus C₂-C₄-Alkoxy, (H₃C-CH₂)-O- (CH₂)₂-O-, (H₃C-CH₂)-O- (CH₂)₂-O-(CH₂)₂-O- und (H₃C-CH₂)-O- (CH₂)₂-O-(CH₂)₂-O-(CH₂)₂-O-,
R⁵ für ein Wasserstoffatom steht
und
R⁶ für ein Wasserstoffatom steht
oder ein Stereoisomer, ein Tautomer, ein Hydrat, ein Solvat oder ein Salz davon oder ein Gemisch davon, oder einen Gd³⁺-Komplex einer Verbindung der folgenden Formel (II) : 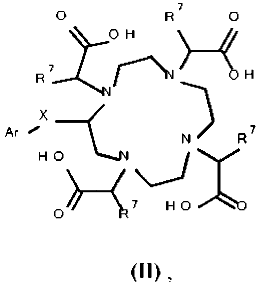
wobei
Ar für eine Gruppe steht, die ausgewählt ist aus 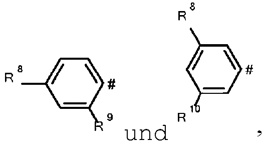
wobei _{#} die Bindung an X ist,
X eine Gruppe ist, die ausgewählt ist aus CH₂, (CH₂)₂, (CH₂)₃, (CH₂)₄ und *- (CH₂)₂-O-CH₂-^{#}, wobei * die Bindung an Ar ist und ^{#} die Bindung an den Essigsäurerest ist,
R⁷ für ein Wasserstoffatom oder eine Gruppe steht, die ausgewählt ist aus C₁-C₃-Alkyl, -CH₂OH, -(CH₂)₂OH und -CH₂OCH₃;
R⁸ für eine Gruppe steht, die ausgewählt ist aus C₂-C₄-Alkoxy, (H₃C-CH₂O)- (CH₂)₂-O-, (H₃C-CH₂O)- (CH₂)₂-O-(CH₂)2-O- und (H₃C-CH₂O)-(CH₂)₂-O-(CH₂)2-O-(CH₂)₂-O-;
R⁹ und R¹⁰ unabhängig voneinander für ein Wasserstoffatom stehen;
oder ein Stereoisomer, ein Tautomer, ein Hydrat, ein Solvat oder ein Salz davon oder ein Gemisch davon.

18. Kit, umfassend ein Kontrastmittel und einen computerlesbaren Programmcode, der bei Ausführung durch eine Verarbeitungseinheit (20) eines Computersystems (1) das Computersystem (1) veranlasst, die folgenden Schritte auszuführen:
(a) Bereitstellen eines vortrainierten bedingten generativen Modells (CGM^{t}), wobei das vortrainierte bedingte generative Modell (CGM^{t}) trainiert wurde, um eine Mehrzahl von Referenzdarstellungen (RR1, RR2, RR3, RR4, RR5, RR6) eines Untersuchungsbereichs einer Mehrzahl von Untersuchungsobjekten zu rekonstruieren,
(b) Bereitstellen einer nativen Darstellung des Untersuchungsbereichs eines neuen Untersuchungsobjekts, wobei die native Darstellung den Untersuchungsbereich des neuen Untersuchungsobjekts ohne Kontrastmittel darstellt,
(c) Bereitstellen einer kontrastverstärkten Darstellung (R1) des Untersuchungsbereichs des neuen Untersuchungsobjekts, wobei die kontrastverstärkte Darstellung (R1) den Untersuchungsbereich des neuen Untersuchungsobjekts nach Verabreichung einer Menge des Kontrastmittels darstellt,
(d) Erzeugen einer oder mehrerer Einbettungen (E1, E2, EC) mindestens teilweise basierend auf der nativen Darstellung und/oder der kontrastverstärkten Darstellung (R1),
(e) Bereitstellen von Anfangsdaten (SD),
(f) Erzeugen einer synthetischen Darstellung (SR) des Untersuchungsbereichs des neuen Untersuchungsobjekts basierend auf den Anfangsdaten (SD) unter Verwendung des vortrainierten bedingten generativen Modells (CGM^{t}) und unter Verwendung der einen oder der mehreren Einbettungen (E1, E2, EC) als Bedingung,
(g) Erzeugen einer transformierten synthetischen Darstellung (SR^{T}) basierend auf der synthetischen Darstellung (SR), wobei die transformierte synthetische Darstellung (SR^{T}) eine kontrastverstärkte Darstellung des Untersuchungsbereichs des neuen Untersuchungsobjekts ist, in dem die Kontrastverstärkung im Vergleich zu der synthetischen Darstellung (SR) reduziert ist,
(h) Quantifizieren von Abweichungen zwischen der transformierten synthetischen Darstellung (SR^{T}) und der kontrastverstärkten Darstellung (R1) des Untersuchungsbereichs des neuen Untersuchungsobjekts,
(i) Reduzieren der Abweichungen durch Modifizieren der Anfangsdaten (SD) und/oder der einen oder der mehreren Einbettungen (E1, E2, EC) und/oder von Parametern des vortrainierten bedingten generativen Modells (CGM^{t}),
(j) ein- oder mehrmaliges Wiederholen der Schritte (f) bis (i),
(k) Ausgeben und/oder Speichern der synthetischen Darstellung (SR) des Untersuchungsbereichs des neuen Untersuchungsobjekts und/oder eines daraus erzeugten synthetischen Bildes des Untersuchungsbereichs des neuen Untersuchungsobjekts und/oder Übertragen der synthetischen Darstellung (SR) und/oder des synthetischen Bildes an ein separates Computersystem.

## Revendications

1. Procédé mis en œuvre par ordinateur comprenant :
(a) l'obtention d'un modèle génératif conditionnel pré-entraîné (CGM^{t}), le modèle génératif conditionnel pré-entraîné (CGM^{t}) ayant été entraîné à reconstruire une pluralité de représentations de référence (RR1, RR2, RR3, RR4, RR5, RR6) d'une zone d'examen d'une pluralité d'objets d'examen,
(b) l'obtention d'une représentation native (R0) de la zone d'examen d'un nouvel objet d'examen, la représentation native (R0) représentant la zone d'examen du nouvel objet d'examen sans agent de contraste,
(c) l'obtention d'une représentation à contraste amélioré (R1) de la zone d'examen du nouvel objet d'examen, la représentation à contraste amélioré (R1) représentant la zone d'examen du nouvel objet d'examen après administration d'une quantité d'un agent de contraste,
(d) la génération d'une ou plusieurs intégrations (E1, E2, EC) au moins partiellement basées sur la représentation native (R0) et/ou la représentation à contraste amélioré (R1),
(e) l'obtention de données de départ (SD),
(f) la génération d'une représentation synthétique (SR) de la zone d'examen du nouvel objet d'examen sur la base des données de départ (SD) au moyen du modèle génératif conditionnel pré-entraîné (CGM^{t}) et au moyen de la ou des intégrations (E1, E2, EC) comme condition,
(g) la génération d'une représentation synthétique transformée (SR^{T}) basée sur la représentation synthétique (SR), la représentation synthétique transformée (SR^{T}) étant une représentation à contraste amélioré de la zone d'examen du nouvel objet d'examen dans laquelle l'amélioration du contraste est réduite par rapport à la représentation synthétique (SR),
(h) la quantification d'écarts entre la représentation synthétique transformée (SR^{T}) et la représentation à contraste amélioré (R1) de la zone d'examen du nouvel objet d'examen,
(i) la réduction des écarts par modification des données de départ (SD) et/ou de la ou des intégrations (E1, E2, EC) et/ou de paramètres du modèle génératif conditionnel pré-entraîné (CGM^{t}),
(j) la répétition des étapes (f) à (i) une ou plusieurs fois,
(k) la restitution et/ou le stockage de la représentation synthétique (SR) de la zone d'examen du nouvel objet d'examen et/ou d'une image synthétique de la zone d'examen du nouvel objet d'examen générée à partir de celle-ci, et/ou la transmission de la représentation synthétique (SR) et/ou de l'image synthétique à un système informatique distinct.

2. Procédé mis en œuvre par ordinateur de la revendication 1, dans lequel le modèle génératif conditionnel pré-entraîné (CGM^{t}) a été pré-entraîné sur des données d'entraînement (TD), les données d'entraînement (TD) comprenant, pour chaque objet d'examen de la pluralité d'objets d'examen, une ou plusieurs représentations de référence (RR1, RR2, RR3, RR4, RR5, RR6) de la zone d'examen de l'objet d'examen, le pré-entraînement comprenant, pour chaque objet d'examen de la pluralité d'objets d'examen :
- la génération d'une ou plusieurs intégrations (E1, E2, EC) basées sur la ou les représentations de référence (RR1, RR2, RR3, RR4, RR5, RR6) de la zone d'examen de l'objet d'examen,
- l'introduction d'une ou plusieurs représentations de référence (RR1, RR2, RR3, RR4, RR5, RR6) de la zone d'examen de l'objet d'examen dans le modèle génératif conditionnel (CGM), et la reconstruction de la représentation ou d'au moins une des représentations de référence (RR1, RR2, RR3, RR4, RR5, RR6) par le modèle génératif conditionnel (CGM) au moyen de la ou des intégrations (E1, E2, EC) comme condition,
- la réception d'une représentation de référence reconstruite (RR1^{R}, RR2^{R}, RR3^{R}, RR4^{R}, RR5^{R}, RR6^{R}) comme résultat du modèle génératif conditionnel (CGM),
- la détermination d'écarts entre la représentation de référence reconstruite (RR1^{R}, RR2^{R}, RR3^{R}, RR4^{R}, RR5^{R}, RR6^{R}) et la représentation ou une des représentations de référence (RR1, RR2, RR3, RR4, RR5, RR6),
- la réduction des écarts par modification de paramètres du modèle génératif conditionnel (CGM).

3. Procédé mis en œuvre par ordinateur de la revendication 1 ou 2, dans lequel chaque objet d'examen est un être humain, la zone d'examen étant ou comprenant un foie, un rein, un cœur, un poumon, un cerveau, un estomac, une vessie, une prostate, un intestin, un œil, un sein, une thyroïde, un pancréas ou un utérus, ou une partie de ceux-ci.

4. Procédé mis en œuvre par ordinateur de l'une quelconque des revendications 1 à 3, dans lequel chaque représentation est une représentation de tomodensitométrie, une représentation de radiographie, une représentation d'imagerie par résonance magnétique, une représentation d'échographie, ou une représentation de tomographie par émission de positons.

5. Procédé mis en œuvre par ordinateur de l'une quelconque des revendications 1 à 4, dans lequel chaque représentation est une représentation dans l'espace réel, dans l'espace fréquentiel ou dans l'espace de projection.

6. Procédé mis en œuvre par ordinateur de l'une quelconque des revendications 1 à 5, dans lequel la génération de la représentation synthétique transformée (SR^{T}) comprend : la réduction de l'amélioration du contraste de la représentation synthétique (SR).

7. Procédé mis en œuvre par ordinateur de l'une quelconque des revendications 1 à 6, dans lequel la génération de la représentation synthétique transformée (SR^{T}) comprend : la réduction de l'amélioration du contraste de la représentation synthétique (SR) de telle sorte que l'amélioration du contraste de la représentation synthétique transformée (SR^{T}) corresponde à l'amélioration du contraste de la représentation à contraste amélioré (R1).

8. Procédé mis en œuvre par ordinateur de l'une quelconque des revendications 1 à 7, dans lequel la génération de la représentation synthétique transformée (SR^{T}) comprend : la réduction de l'amélioration du contraste de la représentation synthétique (SR), ce qui permet de générer une représentation synthétique transformée qui représente la zone d'examen du nouvel objet d'examen après administration de la première quantité d'agent de contraste.

9. Procédé mis en œuvre par ordinateur de l'une quelconque des revendications 1 à 8, dans lequel la génération de la représentation synthétique transformée (SR^{T}) comprend :
- la génération d'une représentation différentielle (DR), la génération de la représentation différentielle (DR) comprenant la soustraction de la représentation native (R0) de la représentation synthétique (SR),
- la génération d'une représentation différentielle atténuée (aDR), la génération de la représentation différentielle atténuée (aDR) comprenant la multiplication de la représentation différentielle (DR) par un facteur d'atténuation,
- l'ajout de la représentation différentielle atténuée (aDR) à la représentation native (R0) ou la soustraction de la représentation différentielle atténuée (aDR) de la représentation synthétique (SR).

10. Procédé mis en œuvre par ordinateur de la revendication 9, dans lequel la génération de la représentation synthétique transformée (SR^{T}) comprend en outre : la multiplication de la représentation différentielle (DR) ou de la représentation différentielle atténuée (aDR) dans l'espace fréquentiel par une fonction de pondération dépendante de la fréquence.

11. Procédé mis en œuvre par ordinateur de l'une quelconque des revendications 1 à 10, dans lequel les étapes (f) à (i) sont répétées jusqu'à ce qu'un critère d'arrêt soit atteint, le critère d'arrêt comprenant : un nombre maximal prédéfini d'étapes a été effectué, les écarts entre la représentation synthétique transformée (SR^{T}) et la représentation à contraste amélioré (R1) ne peuvent plus être réduits en modifiant les données de départ (SD) et/ou la ou les intégrations (E1, E2, EC) et/ou les paramètres du modèle, un minimum prédéfini d'une fonction de perte (LF) est atteint, et/ou une valeur extrême d'une autre valeur de performance est atteinte.

12. Procédé mis en œuvre par ordinateur de l'une quelconque des revendications 1 à 11, dans lequel le modèle génératif conditionnel pré-entraîné (CGM^{t}) est ou comprend un modèle de diffusion (DM^{t}) ou une partie de celui-ci.

13. Procédé mis en œuvre par ordinateur de l'une quelconque des revendications 1 à 12, dans lequel la ou les intégrations (E1, E2, EC) sont générées au moyen d'un ou plusieurs encodeurs (E), le ou les encodeurs (E) comprenant un ou plusieurs transformateurs et/ou réseaux neuronaux convolutifs.

14. Système informatique comprenant :
une unité de traitement (20) ; et
une mémoire (50) stockant un programme informatique (60) conçu pour effectuer, lors de son exécution par l'unité de traitement (20), une opération, l'opération comprenant :
(a) l'obtention d'un modèle génératif conditionnel pré-entraîné (CGM^{t}), le modèle génératif conditionnel pré-entraîné (CGM^{t}) ayant été entraîné à reconstruire une pluralité de représentations de référence (RR1, RR2, RR3, RR4, RR5, RR6) d'une zone d'examen d'une pluralité d'objets d'examen,
(b) l'obtention d'une représentation native (R0) de la zone d'examen d'un nouvel objet d'examen, la représentation native (R0) représentant la zone d'examen du nouvel objet d'examen sans agent de contraste,
(c) l'obtention d'une représentation à contraste amélioré (R1) de la zone d'examen du nouvel objet d'examen, la représentation à contraste amélioré (R1) représentant la zone d'examen du nouvel objet d'examen après administration d'une quantité d'un agent de contraste,
(d) la génération d'une ou plusieurs intégrations (E1, E2, EC) au moins partiellement basées sur la représentation native (R0) et/ou la représentation à contraste amélioré (R1),
(e) l'obtention de données de départ (SD),
(f) la génération d'une représentation synthétique (SR) de la zone d'examen du nouvel objet d'examen sur la base des données de départ (SD) au moyen du modèle génératif conditionnel pré-entraîné (CGM^{t}) et au moyen de la ou des intégrations (E1, E2, EC) comme condition,
(g) la génération d'une représentation synthétique transformée (SR^{T}) basée sur la représentation synthétique (SR), la représentation synthétique transformée (SR^{T}) étant une représentation à contraste amélioré de la zone d'examen du nouvel objet d'examen dans laquelle l'amélioration du contraste est réduite par rapport à la représentation synthétique (SR),
(h) la quantification d'écarts entre la représentation synthétique transformée (SR^{T}) et la représentation à contraste amélioré (R1) de la zone d'examen du nouvel objet d'examen,
(i) la réduction des écarts par modification des données de départ (SD) et/ou de la ou des intégrations (E1, E2, EC) et/ou de paramètres du modèle génératif conditionnel pré-entraîné (CGM^{t}),
(j) la répétition des étapes (f) à (i) une ou plusieurs fois,
(k) la restitution et/ou le stockage de la représentation synthétique (SR) de la zone d'examen du nouvel objet d'examen et/ou d'une image synthétique de la zone d'examen du nouvel objet d'examen générée à partir de celle-ci, et/ou la transmission de la représentation synthétique (SR) et/ou de l'image synthétique à un système informatique distinct.

15. Support de stockage non transitoire lisible par ordinateur sur lequel sont stockées des instructions logicielles qui, lors de leur exécution par une unité de traitement (20) d'un système informatique (1), amènent le système informatique (1) à effectuer les étapes suivantes :
(a) obtention d'un modèle génératif conditionnel pré-entraîné (CGM^{t}), le modèle génératif conditionnel pré-entraîné (CGM^{t}) ayant été entraîné à reconstruire une pluralité de représentations de référence (RR1, RR2, RR3, RR4, RR5, RR6) d'une zone d'examen d'une pluralité d'objets d'examen,
(b) obtention d'une représentation native (R0) de la zone d'examen d'un nouvel objet d'examen, la représentation native (R0) représentant la zone d'examen du nouvel objet d'examen sans agent de contraste,
(c) obtention d'une représentation à contraste amélioré (R1) de la zone d'examen du nouvel objet d'examen, la représentation à contraste amélioré (R1) représentant la zone d'examen du nouvel objet d'examen après administration d'une quantité d'un agent de contraste,
(d) génération d'une ou plusieurs intégrations (E1, E2, EC) au moins partiellement basées sur la représentation native (R0) et/ou la représentation à contraste amélioré (R1),
(e) obtention de données de départ (SD),
(f) génération d'une représentation synthétique (SR) de la zone d'examen du nouvel objet d'examen sur la base des données de départ (SD) au moyen du modèle génératif conditionnel pré-entraîné (CGM^{t}) et au moyen de la ou des intégrations (E1, E2, EC) comme condition,
(g) génération d'une représentation synthétique transformée (SR^{T}) basée sur la représentation synthétique (SR), la représentation synthétique transformée (SR^{T}) étant une représentation à contraste amélioré de la zone d'examen du nouvel objet d'examen dans laquelle l'amélioration du contraste est réduite par rapport à la représentation synthétique (SR),
(h) quantification d'écarts entre la représentation synthétique transformée (SR^{T}) et la représentation à contraste amélioré (R1) de la zone d'examen du nouvel objet d'examen,
(i) réduction des écarts par modification des données de départ (SD) et/ou de la ou des intégrations (E1, E2, EC) et/ou de paramètres du modèle génératif conditionnel pré-entraîné (CGM^{t}),
(j) répétition des étapes (f) à (i) une ou plusieurs fois,
(k) restitution et/ou stockage de la représentation synthétique (SR) de la zone d'examen du nouvel objet d'examen et/ou d'une image synthétique de la zone d'examen du nouvel objet d'examen générée à partir de celle-ci, et/ou transmission de la représentation synthétique (SR) et/ou de l'image synthétique à un système informatique distinct.

16. Utilisation d'un agent de contraste dans un examen d'une zone d'examen d'un nouvel objet d'examen, l'examen comprenant :
(a) l'obtention d'un modèle génératif conditionnel pré-entraîné (CGM^{t}), le modèle génératif conditionnel pré-entraîné (CGM^{t}) ayant été entraîné à reconstruire une pluralité de représentations de référence (RR1, RR2, RR3, RR4, RR5, RR6) d'une zone d'examen d'une pluralité d'objets d'examen,
(b) la génération d'une représentation native (R0) de la zone d'examen du nouvel objet d'examen, la représentation native (R0) représentant la zone d'examen du nouvel objet d'examen sans agent de contraste,
(c) la génération d'une représentation à contraste amélioré (R1) de la zone d'examen du nouvel objet d'examen, la représentation à contraste amélioré (R1) représentant la zone d'examen du nouvel objet d'examen après administration d'une quantité de l'agent de contraste,
(d) la génération d'une ou plusieurs intégrations (E1, E2, EC) au moins partiellement basées sur la représentation native (R0) et/ou la représentation à contraste amélioré (R1),
(e) l'obtention de données de départ (SD),
(f) la génération d'une représentation synthétique (SR) de la zone d'examen du nouvel objet d'examen sur la base des données de départ (SD) au moyen du modèle génératif conditionnel pré-entraîné (CGM^{t}) et au moyen de la ou des intégrations (E1, E2, EC) comme condition,
(g) la génération d'une représentation synthétique transformée (SR^{T}) basée sur la représentation synthétique (SR), la représentation synthétique transformée (SR^{T}) étant une représentation à contraste amélioré de la zone d'examen du nouvel objet d'examen dans laquelle l'amélioration du contraste est réduite par rapport à la représentation synthétique (SR),
(h) la quantification d'écarts entre la représentation synthétique transformée (SR^{T}) et la représentation à contraste amélioré (R1) de la zone d'examen du nouvel objet d'examen,
(i) la réduction des écarts par modification des données de départ (SD) et/ou de la ou des intégrations (E1, E2, EC) et/ou de paramètres du modèle génératif conditionnel pré-entraîné (CGM^{t}),
(j) la répétition des étapes (f) à (i) une ou plusieurs fois,
(k) la restitution et/ou le stockage de la représentation synthétique (SR) de la zone d'examen du nouvel objet d'examen et/ou d'une image synthétique de la zone d'examen du nouvel objet d'examen générée à partir de celle-ci, et/ou la transmission de la représentation synthétique (SR) et/ou de l'image synthétique à un système informatique distinct.

17. Utilisation de la revendication 16, dans laquelle l'agent de contraste est ou comprend :
le 2,2',2"-(10-{1-carboxy-2-[2-(4-éthoxyphényl)éthoxy]éthyl}-1,4,7,10-tétraazacyclododécane-1,4,7-triyl)triacétate de gadolinium,
le 2,2',2"-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
le 2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
le (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
le 2,2',2"-{10-[(1S)-4-(4-butoxyphényl)-1-carboxybutyl]-1,4,7,10-tétraazacyclododécane-1,4,7-triyl}triacétate de gadolinium,
le 2,2',2"-((2S)-10-(carboxyméthyl)-2-[4-(2-éthoxyéthoxy)benzyl]-1,4,7,10-tétraazacyclododécane-1,4,7-triyl}triacétate de gadolinium,
le 2,2',2"-[10-(carboxyméthyl)-2-(4-éthoxybenzyl)-1,4,7,10-tétraazacyclododécane-1,4,7-triyl]triacétate de gadolinium.
l'hydrate de 5,8-bis(carboxylatométhyl)-2-[2-(méthylamino)-2-oxoéthyl]-10-oxo-2,5,8,11-tétraazadodécane-1-carboxylate de gadolinium(III),
le 2-[4-(2-hydroxypropyl)-7,10-bis(2-oxido-2-oxoéthyl)-1,4,7,10-tétrazacyclododéc-1-yl]acétate de gadolinium(III),
le 2,2',2"-(10-((2R,3S)-1,3,4-trihydroxybutan-2-yl)-1,4,7,10-tétraazacyclododécane-1,4,7-triyl)triacétate de gadolinium(III),
l'acide 2-[4,7,10-tris(carboxyméthyl)-1,4,7,10-tétrazacyclododéc-1-yl]acétique de gadolinium(III),
l'acide éthoxybenzyldiéthylènetriaminepentaacétique de gadolinium(III),
le 2-[3,9-bis[1-carboxylato-4-(2,3-dihydroxypropylamino)-4-oxobutyl]-3,6,9,15-tétrazabicyclo[9.3.1]pentadéca-1(15),11,13-trién-6-yl]-5-(2,3-dihydroxypropylamino)-5-oxopentanoate de gadolinium,
le [(±)-4-carboxy-5,8,11-tris(carboxyméthyl)-1-phényl-2-oxa-5,8,11-triazatridécan-13-oato(5-)]gadolinate(2-) de dihydrogène,
le [4,10-bis(carboxylatométhyl)-7-{3,6,12,15-tétraoxo-16-[4,7,10-tris-(carboxylatométhyl)-1,4,7,10-tétraazacyclododécan-1-yl]-9,9-bis({[({2-[4,7,10-tris-(carboxylatométhyl)-1,4,7,10-tétraazacyclododécan-1-yl]propanoyl}amino)acétyl]-amino}méthyl)-4,7,11,14-tétraazahepta-décan-2-yl}-1,4,7,10-tétraazacyclododécan-1-yl]acétate de tétragadolinium,
un complexe de Gd³⁺ d'un composé de formule (I) 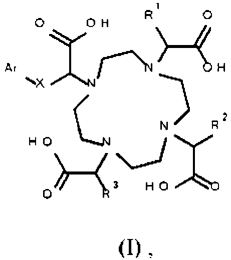
où
Ar représente un groupe choisi parmi 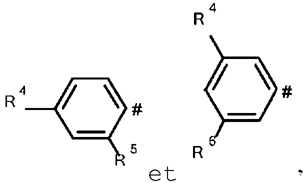
où # est la liaison à X,
X représente un groupe choisi parmi
CH₂, (CH₂)₂, (CH₂)₃, (CH₂)₄ et *- (CH₂)₂-O-CH₂-^{#},
où * est la liaison à Ar et ^{#} est la liaison au résidu acide acétique,
R¹, R² et R³ représentent chacun indépendamment un atome d'hydrogène ou un groupe choisi parmi un alkyle en C₁-C₃, -CH₂OH, - (CH₂)₂OH et -CH₂OCH₃,
R⁴ représente un groupe choisi parmi un alcoxy en C₂-C₄, (H₃C-CH₂)-O-(CH₂)₂-O-, (H₃C-CH₂)-O-(CH₂)₂-O-(CH₂)₂-O- et (H₃C-CH₂)-O-(CH₂)₂-O-(CH₂)₂-O-(CH₂)₂-O-,
R⁵ représente un atome d'hydrogène,
et
R⁶ représente un atome d'hydrogène,
ou un stéréoisomère, tautomère, hydrate, solvate ou sel de celui-ci, ou un mélange de ceux-ci, ou
un complexe de Gd³⁺ d'un composé de formule (II) 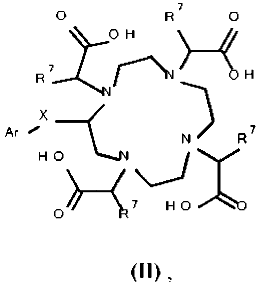
où
Ar représente un groupe choisi parmi 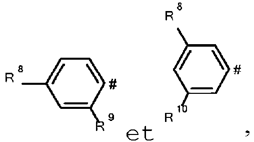
où # est la liaison à X,
X représente un groupe choisi parmi CH₂, (CH₂)₂, (CH₂)₃, (CH₂)₄ et *-(CH₂)₂-O-CH₂-^{#}, où * est la liaison à Ar et ^{#} est la liaison au résidu acide acétique,
R⁷ représente un atome d'hydrogène ou un groupe choisi parmi un alkyle en C₁-C₃, -CH₂OH, -(CH₂)₂OH et -CH₂OCH₃ ;
R⁸ représente un groupe choisi parmi un alcoxy en C₂-C₄, (H₃C-CH₂O)- (CH₂)₂-O-, (H₃C-CH₂O)-(CH₂)₂-O-(CH₂)₂-O- et (H₃C-CH₂O)-(CH₂)2-O-(CH₂)2-O-(CH₂)2-O- ;
R⁹ et R¹⁰ représentent indépendamment un atome d'hydrogène ;
ou un stéréoisomère, tautomère, hydrate, solvate ou sel de celui-ci, ou un mélange de ceux-ci.

18. Kit comprenant un agent de contraste et un code de programme lisible par ordinateur qui, lors de son exécution par une unité de traitement (20) d'un système informatique (1), amène le système informatique (1) à exécuter les étapes suivantes :
(a) obtention d'un modèle génératif conditionnel pré-entraîné (CGM^{t}), le modèle génératif conditionnel pré-entraîné (CGM^{t}) ayant été entraîné à reconstruire une pluralité de représentations de référence (RR1, RR2, RR3, RR4, RR5, RR6) d'une zone d'examen d'une pluralité d'objets d'examen,
(b) obtention d'une représentation native de la zone d'examen d'un nouvel objet d'examen, la représentation native représentant la zone d'examen du nouvel objet d'examen sans agent de contraste,
(c) obtention d'une représentation à contraste amélioré (R1) de la zone d'examen du nouvel objet d'examen, la représentation à contraste amélioré (R1) représentant la zone d'examen du nouvel objet d'examen après administration d'une quantité de l'agent de contraste,
(d) génération d'une ou plusieurs intégrations (E1, E2, EC) au moins partiellement basées sur la représentation native et/ou la représentation à contraste amélioré (R1),
(e) obtention de données de départ (SD),
(f) génération d'une représentation synthétique (SR) de la zone d'examen du nouvel objet d'examen sur la base des données de départ (SD) au moyen du modèle génératif conditionnel pré-entraîné (CGM^{t}) et au moyen de la ou des intégrations (E1, E2, EC) comme condition,
(g) génération d'une représentation synthétique transformée (SR^{T}) basée sur la représentation synthétique (SR), la représentation synthétique transformée (SR^{T}) étant une représentation à contraste amélioré de la zone d'examen du nouvel objet d'examen dans laquelle l'amélioration du contraste est réduite par rapport à la représentation synthétique (SR),
(h) quantification d'écarts entre la représentation synthétique transformée (SR^{T}) et la représentation à contraste amélioré (R1) de la zone d'examen du nouvel objet d'examen,
(i) réduction des écarts par modification des données de départ (SD) et/ou de la ou des intégrations (E1, E2, EC) et/ou de paramètres du modèle génératif conditionnel pré-entraîné (CGM^{t}),
(j) répétition des étapes (f) à (i) une ou plusieurs fois,
(k) restitution et/ou stockage de la représentation synthétique (SR) de la zone d'examen du nouvel objet d'examen et/ou d'une image synthétique de la zone d'examen du nouvel objet d'examen générée à partir de celle-ci, et/ou transmission de la représentation synthétique (SR) et/ou de l'image synthétique à un système informatique distinct.
